# EUROPEAN PATENT SPECIFICATION

(11) **EP 3 877 753 B1**
(45) Date of publication and mention of the grant of the patent: **08.10.2025**
(21) Application number: 19881202.6
(22) Date of filing: 06.11.2019
(51) Int. Cl.: G01N 21/64, G01N 33/53, C12Q 1/02

(54) **NON-ENZYMATIC GLOW ASSAYS**
NICHTENZYMATISCHE GLOW-TESTS
DOSAGES PAR LUMINESCENCE NON ENZYMATIQUES

(30) Priority: 07.11.2018 US 201862756801 P
(43) Date of publication of application: 15.09.2021
(73) Proprietor: University of Houston System, Houston, TX 77204-2015 (US)
(72) Inventor: VU, Binh, V., Houston, TX 77204 (US); WILLSON, Richard, C., Houston, TX 77204 (US)
(74) Representative: Rückerl, Florian
(86) International application number: PCT/US2019/059953
(87) International publication number: WO 2020/097138

(56) References cited:
- WO-A2-00/44719
- CN-A- 106 093 408
- US-A1- 2003 021 790
- US-A1- 2008 261 239
- US-A1- 2009 130 771
- US-A1- 2009 298 048
- US-A1- 2013 001 485
- US-A1- 2013 084 652
- US-A1- 2018 031 484
- EGLEN RICHARD M ET AL: "The use of AlphaScreen technology in HTS: current status", CURRENT CHEMICAL GENOMICS, BENTHAM OPEN, NL, vol. 1, 1 January 2008 (2008-01-01), pages 2 - 10, XP008176468, ISSN: 1875-3973, [retrieved on 20080225], DOI: 10.2174/1875397300801010002
- HOU CHANGJIANG ET AL: "Donor/acceptor nanoparticle pair-based singlet oxygen channeling homogenous chemiluminescence immunoassay for quantitative determination of bisphenol A", ANALYTICAL AND BIOANALYTICAL CHEMISTRY, SPRINGER BERLIN HEIDELBERG, BERLIN/HEIDELBERG, vol. 408, no. 30, 29 April 2016 (2016-04-29), pages 8795 - 8804, XP036110427, ISSN: 1618-2642, [retrieved on 20160429], DOI: 10.1007/S00216-016-9584-Y
- MECHALY ADVA ET AL: "A novel homogeneous immunoassay for anthrax detection based on the AlphaLISA method: detection of B. anthracis spores and protective antigen (PA) in complex samples", ANALYTICAL AND BIOANALYTICAL CHEMISTRY, vol. 405, no. 12, 1 May 2013 (2013-05-01), Berlin/Heidelberg, pages 3965 - 3972, XP055843581, ISSN: 1618-2642, Retrieved from the Internet <URL:https://link.springer.com/content/pdf/10.1007/s00216-013-6752-1.pdf> DOI: 10.1007/s00216-013-6752-1
- ALBA F J ET AL: "NONENZYMATIC CHEMILUMINESCENT DETECTION AND QUANTITATION OF TOTAL PROTEIN ON WESTERN AND SLOT BLOTS ALLOWING SUBSEQUENT IMMUNODETECTION AND SEQUENCING", ELECTROPHORESIS, VERLAG CHEMIE, HOBOKEN, USA, vol. 18, no. 11, 1 October 1997 (1997-10-01), pages 1960 - 1966, XP009076701, ISSN: 0173-0835, DOI: 10.1002/ELPS.1150181114
- FILANOSKI BRIAN ET AL: "Non-enzymatic aqueous peroxyoxalate chemiluminescence immune detection using a CCD camera and a CMOS device", LUMINESCENCE: THE JOURNAL OF BIOLOGICAL AND CHEMICAL LUMINESCENCE, vol. 23, no. 5, 1 October 2008 (2008-10-01), GB, pages 296 - 302, XP55933625, ISSN: 1522-7235, DOI: 10.1002/bio.1033
- EGLEN R. M. ET AL.: "The use of AlphaScreen technology in HTS: current status", CURRENT CHEMICAL GENOMICS, vol. 1, 2008, pages 2 - 10, XP008176468, DOI: 10.2174/1875397300801010002
- HANANYA N. ET AL.: "A Highly Efficient Chemiluminescence Probe for the Detection of Singlet Oxygen in Living Cells", ANGEWANDTE CHEMIE -INTERNATIONAL EDITION, vol. 56, no. 39, 2017, pages 11793 - 11796, XP055704718
- SHU X. ET AL.: "A Genetically Encoded Tag for Correlated Light and Electron Microscopy of Intact Cells, Tissues, and Organisms", PLOS BIOLOGY, vol. 9, no. 4, 2011, pages 1 - 10, XP055704722
- CHEN H. ET AL.: "Nanoparticle-Based Proximity Ligation Assay for Ultrasensitive, Quantitative Detection of Protein Biomarkers", ACS APPLIED MATERIALS & INTERFACES, vol. 10, no. 38, 26 September 2018 (2018-09-26), pages 31845 - 31849, XP055704723

## Description

### BACKGROUND

### I. Field

The present disclosure relates to the fields of diagnostics, immunoassays, and medicine. More particularly, the disclosure relates to an improved method of performing a lateral or vertical flow light-based assay, especially ligand binding assay, in which light is emitted from moieties stimulated by stimulating moieties.

### II. Related Art

Lateral flow and other point-of-care diagnostics are extremely valuable in allowing untrained people to perform rapid diagnosis of pregnancy, overdose, autoimmune disease flares, and infection. They require no equipment and are very inexpensive; the home pregnancy test is the best-known example. There are two general weaknesses of current test formats. Their sensitivity is limited to about five parts per billion, and they are not very quantitative.

Many assays and biomedical diagnostics involve the transduction of the presence or concentration of an analyte into a detectable signal. This signal can be electrochemical, color formation, a generated reporter molecule, polarization, or other readout. One signal which is particularly valuable is the emission of light. Light emission can be by chemiluminescence, bioluminescence, electrochemiluminescence, phosphorescence or fluorescence. Suppression or blocking of light in the presence of an analyte can also serve to report its presence.

An exemplary assay is described in US patent application US2013/0084652 A1 which discloses homogeneous assays for detecting the presence of substances by means of specific binding pair reactions. using a chemiluminescent compound connected with a first specific binding partner, a sensitizer compound conjugated to a second specific binding partner, and a noise modulation agent, an enhancer, in a solution phase. As a result of the specific binding partners binding due to the analyte, a sensitizer is brought into operable proximity to a chemiluminescent compound so that it is effective to produce a metastable species in proximity to the chemiluminescent compound. Reaction of the metastable species with the chemiluminescent compound results in the generation of light. In contrast to this assay method, the method according to the present invention exhibits a separation step and does not require a noise modulation agent.

Among the most common assay or diagnostic formats is the ligand binding assay, in which a molecular recognition element such as an antibody, aptamer, DNA probe, or lectin binds to the analyte or a competitor, and this binding modulates the position, activity or retention of a light-generating moiety. Ligand binding assays are commonly conducted in microtiter plates or in lateral flow or vertical flow membranes, but also can take place in tubes, on flat surfaces, or in flow injection analysis apparatus or microfluidic devices, among others.

A prominent example is a lateral flow sandwich immunoassay in which capture antibodies on a lateral flow membrane bind target analyte delivered to them in flow along the length of the membrane. A light-emitting moiety binds in turn to the target analyte, via a molecular recognition element with which it is physically associated. The light emitter which is bound to the target is associated with the molecular recognition element by covalent or noncovalent interactions. This association can be by direct chemical coupling, or both light emitter and recognition element may be associated with a polymer or particle.

### SUMMARY

This disclosure concerns new formats for light-based assays, especially ligand binding assays, in which light is emitted from moieties stimulated by stimulating moieties. Stimulating moieties provide chemiexcitation molecules. Of particular interest are assays conducted in a lateral-flow immunoassay format, in which either the light emitting moiety or the exciting moiety is physically associated with a molecular recognition element.

Thus, there is provided a method of performing a lateral or vertical flow assay comprising (a) providing a fluid sample comprising an analyte of interest; (b) contacting said sample with a membrane or filter comprising a first reagent comprising a molecular recognition element that binds to said analyte; (c) contacting said sample with a second reagent that:
(i) binds to said analyte in the presence of said first reagent; or
(ii) binds to said first reagent;

wherein said second reagent contains a chemiexcitation emitter; (d) removing unbound first and second reagents; (e) introducing into said sample a chemiexcitation stimulator; and (f) measuring the production of light from said emitter,
wherein the chemiexcitation stimulator is peroxyoxalate chemiluminescence resulting from the energy transferred from the reaction of peroxide and peroxyoxalate ester to the chemiluminescent emitter, and
wherein the second reagent is in the form of a particle coated or functionalized with the chemiexcitation emitter and the recognition element.

The chemiexcitation emitter may be rhodamine 101, rhodamine B, anthracene, 2-methoxy-2,4-diphenyl-3(2H)-furanone (MDPF), ethidium bromide, propidium iodide, the bis-intercalating dyes ethidium homodimer-1, benzoxazolium-4-pyridinium dimer-1 and benzoxazolium-4-quinolinium dimer-1, Texas Red, ROX, SYPRO Ruby, 2-methoxy-2,4-diphenyl-3(2H)-furanone (MDPF), fluorescent dye from Alexa Fluor family, and other derivatives from rhodamine, terrylene, perylene, and phenylethylanthracene, The chemiluminescent compound may include bis(2,4,6-trichlorophenyl) oxalate (TCPO), bis(2,4,5-trichlorophenyl)oxalate (2,4,5 TCPO), bis(1,1,1,3,3,3-hexafluoro-2-propyl)oxalate, bis[1-(1H)-2-pyridonyl]glyoxal, bis(2,4,5-trichlorophenyl-6-carbopentoxyphenyl)oxalate(CPPO),Bis(2,4-dinitro-6-methylphenyl)oxalate(DNNPO),bis(2,14-dinitrophenyl)oxalate(DNPO),bis(3-trifluoromethyi4-nitrophenyl)oxalate (TFMNPO), divanillyl oxalate, a pyridonylglyoxal reagent such as Bis(1-[1H]-5-nitro-2-pyridonyl)glyoxal (NPG), an oxalic anhydride reagent such as triphenylacetic oxalic anhydride (TPAOA), acetic oxalic anhydride (AOA), or a watersoluble peroxyoxalate reagents such as peroxyoxamide, 2,2'-Oxalylbis[(trifluoromethanesulfonyl)imino]ethylene-bis(N-methylpyridinium)trifluoromethanesulfonate, and 2,2'-Oxalylbis[(trifluoromethanesulfonyl)imino]ethylene-bis(N-methylpyridinium)tetrafluoroborate.

The particle may have a diameter of less than 1 micron, or less than 400 nanometers. The particle may be a bacteriophage, a polymeric nanoparticle, a metal oxide nanoparticle, a metalloid oxide nanoparticle, a carbohydrate nanoparticle, a porous particle, a quantum dot, a phosphor, a fluorescent material, or a particle comprising light emitters. The source of singlet oxygen or the chemiexcitation stimulator may be activated by light, electrons, an electric field, photons, or combinations thereof. The molecular recognition element may comprise an aptamer, a protein, a carbohydrate or a nucleic acid, such as a protein beacon, analyte-sensitive fluorescent protein, allosteric molecular beacon, hairpin peptide beacon, molecular aptamer beacon, quantum dot aptamer beacon, molecular beacon aptamer, allosteric fluorescent protein biosensor, or molecular switch sensor. The first and/or second reagent may comprise a polymer to which the chemiexcitation emitter is linked. The polymer may be polyethylene glycol, a carbohydrate and a zwitterion, or a dendrimer.

The method may further comprise introducing a catalyst into the sample or subjecting the sample to heat. The catalyst may be sodium salicylate, tripropylamine, triethylamine, sodium acetate, tetrabutylammonium perchlorate, benzyltrimethylammonium hydroxide, oxalic, trichloroacetic, or picric acid.

The chemiluminescent emitter may comprise at least one chemiluminescent particle and at least one magnetic particle or layer of magnetic material. A shell may encapsulate the at least one chemiluminescent particle and the at least one magnetic particle or layer of magnetic material. The method may further comprise concentrating the magnetic chemiluminescent emitter by applying a magnetic field.

The assay may be is a quantitative assay. The sample may be applied to a support comprising said first reagent, such as a porous membrane including nitrocellulose, glass fibers, cotton fibers, a microfluidic chip, paper, a membrane, a microplate, a microbubble, or a transparent surface. The method may be a surface-bound assay, a flow-through assay, a buoyancy assay, or a magnetic assay. Target analytes can include pathogens and parts of pathogens, proteins, nucleic acids, biomarkers, hormones, chemicals, xenobiotics, and environmental and food contaminants, among many other examples.

A particular assay of interest is a later flow assay (LFA). The assay LFA may employ flow through or along a membrane. The membrane may be disposed on an impermeable backing pad. The signal may be localized to one or more specific regions of the membrane. The membrane may be disposed in a cartridge, such as a disposable cartridge.

The assays may employ readout by smartphone and/or wireless transmission of the results. The dimension of the membrane may be relatively small, less than 10 cm² down to about 10 mm² or even smaller. The assay may be performed in the absence of blotting, may employ vacuum transfer, may have a short completion time (e.g., below 30 min sample-to-answer).

The use of the word "a" or "an" when used in conjunction with the term "comprising" in the claims and/or the specification may mean "one," but it is also consistent with the meaning of "one or more," "at least one," and "one or more than one." The word "about" means plus or minus 5% of the stated number.

### BRIEF DESCRIPTION OF THE DRAWINGS

The following drawings form part of the present specification and are included to further demonstrate certain aspects of the present disclosure. The disclosure may be better understood by reference to one or more of these drawings in combination with the detailed description of specific embodiments presented herein.

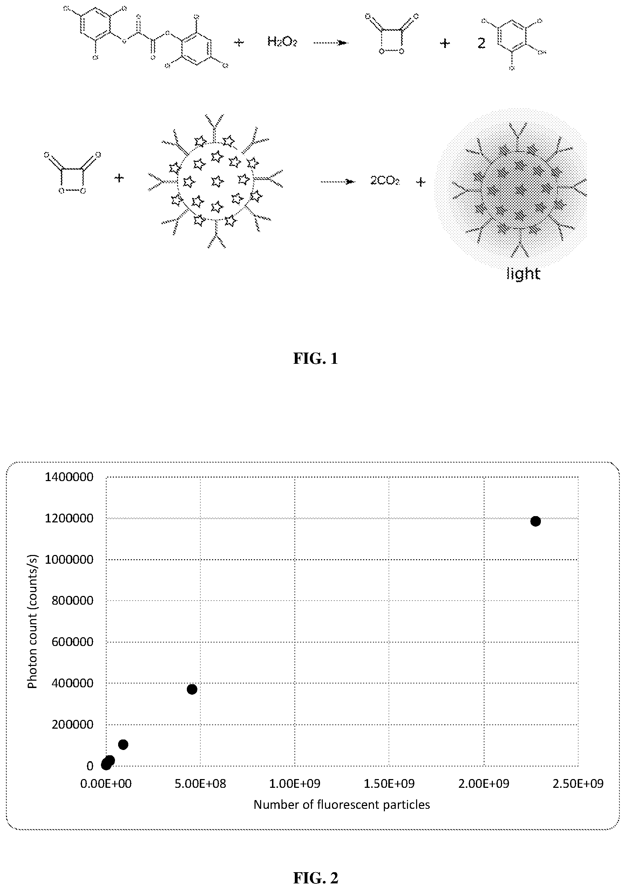

**FIG. 1** - A chemiexcitation method of to excite chemiluminescent reporter particles carrying detection antibody and fluorescer with peroxyoxalate chemistry.
**FIG. 2** **-** Luminescent signal versus the number of fluorescent particles.

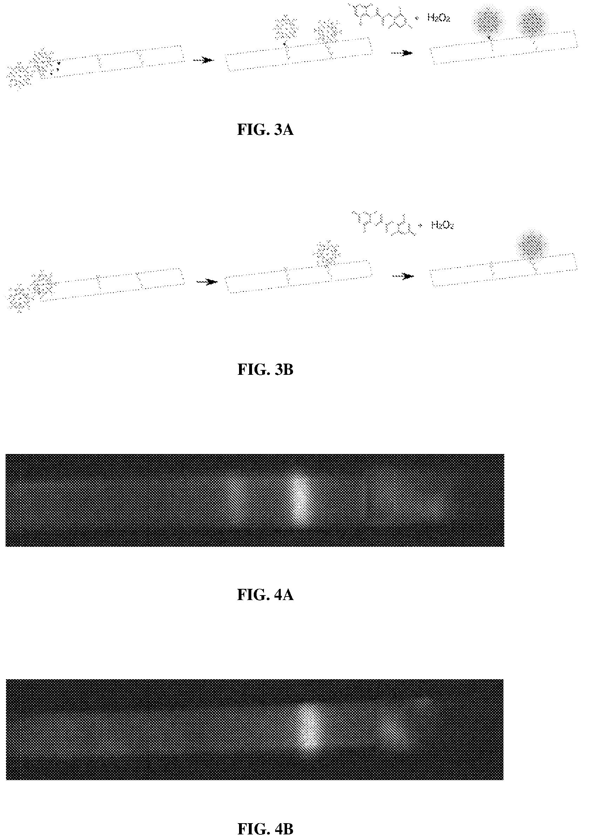

**FIGS. 3A-B** - (FIG. 3A) In the presence of analyte. (FIG. 3B) In the absence of analyte.
**FIGS. 4A-B** - (FIG. 4A) Image of LFA strip when analyte is present. (FIG. 4B) Image of LFA strip when analyte is absent.

### DETAILED DESCRIPTION

### I. Assay Features

The present disclosure concerns new formats for light-based assays, especially ligand binding assays, in which light is emitted from moieties stimulated by stimulating moieties. Stimulating moieties produce by chemiexcitation molecules. Of particular interest are assays conducted in a lateral-flow immunoassay format, in which either the light emitting moiety or the exciting moiety is physically associated with a molecular recognition element.

The claimed methods involve peroxyoxalate chemiluminescence. The chemiexcitation method comprises of a peroxide, a peroxyoxalate ester, a catalyst or enhancer, and fluorescer conjugated to a recognition element function as a chemiluminescent reporter; in which the presence of the chemiluminescent reporter light will be generated by the energy transferred from the reaction of peroxide and peroxyoxalate ester to the chemiluminescent reporter. The chemiluminescent intensity corresponding to the amount of chemiluminescent reporter which can be used to quantify the analyte.

The chemiluminescent reporter is in the form of particles coated or functionalized with both fluorescer and recognition element. Particles preferred in this application include particles with diameter less than 1 micron, or less than 400 nanometers, nanoparticles, bacteriophage, polymeric nanoparticles, metal oxide nanoparticles, metalloid oxide nanoparticles, carbohydrate nanoparticles, porous particles, quantum dots, phosphors, fluorescent materials, and particles comprising light emitters. To the emitting moiety is applied the stimulating moiety, to produce light emission to report the binding even and so the presence of the target analyte. It also is possible for the molecular recognition element to be associated with a stimulating moiety instead of an emitting moiety, with the emitting moiety being applied separately. Polymers preferred in this application include polyethylene glycol, carbohydrates and zwitterions, dendrimers.

Particular stimulating moieties for singlet oxygen disclosed but not claimed, are illuminated phthalocyanine and Tsien's "miniSOG", a small (106-residue) fluorescent flavoprotein which generates singlet oxygen when illuminated by blue light (Shu et al., PLOS Biology, 9(4): e1001041, 2011; doi.org/10.1371/journal.pbio.1001041).

Particular emitting moieties for singlet oxygen disclosed but not claimed, are Hananya's SOCL probe, and Perkin Elmer AlphaLisa and AlphaScreen particles containing thioxene, anthracene, and rubrene in the former case, and thioxene and a europium chelate in the latter.

Particular stimulating moieties for chemiexcitation disclosed but not claimed are the chemistry of cold light sticks, oxalyl chloride and hydrogen peroxide, oxalate esters, bis(2,4,6-trichlorophenyl)oxalate (TCPO) and hydrogen peroxide. In addition to hydrogen peroxide, other major classes of peroxides are can also be used, some of which conveniently are solids. In some embodiments, the peroxide is a metal peroxide, which is metal-containing compound with ionic or covalently bonded peroxide (O₂²⁻) group such as barium peroxide, sodium peroxide, calcium peroxide, lithium peroxide, lithium peroxide, magnesium peroxide, nickel peroxide, nickel(II) peroxide hydrate, zinc peroxide, or strontium peroxide. In some embodiments, the peroxide is an organic peroxide such as tert-butylhydroperoxide, 2.4-pentanedione peroxide, 2-butanone peroxide, benzoyl peroxide, dicumyl peroxide, di-tert-amyl peroxide, tert-butyl peroxide, or lauroyl peroxide. In some embodiments, the peroxide is a main group peroxides such as potassium peroxydisulfate, ammonium persulfate, potassium peroxodisulfate, potassium persulfate, sodium perborate tetrahydrate, or sodium persulfate. In some embodiments, the peroxide is a peroxy acid such as peroxymonosulfuric acid and peracetic acid.

Stimulating moieties for chemiexcitation may be contacted with emitting moieties by pouring or dripping, or by wicking or permeation along or through a permeable material, or dissolution of effusion from a source, or by blending of streams. They may be blended, prepared or formulated shortly before use. They or their components may be stored in bottles or dropper bottles or blisters associated with assay materials such as a lateral flow membrane, e.g. in a kit or assembled assay unit.

Particular emitting moieties for chemiexcitation are rhodamine 101, rhodamine B, anthracene, 2-methoxy-2,4-diphenyl-3(2H)-furanone (MDPF), ethidium bromide, propidium iodide, the bis-intercalating dyes ethidium homodimer-1, benzoxazolium-4-pyridinium dimer-1 and benzoxazolium-4-quinolinium dimer-1, Texas Red, ROX, SYPRO Ruby, 2-methoxy-2,4-diphenyl-3(2H)-furanone (MDPF), fluorescent dye from Alexa Fluor family, and other derivatives from rhodamine, terrylene, perylene, and phenylethylanthracene, and natural derived dyes such as chlorophyll, green fluorescent protein (GFP), and other fluorescent proteins.

In some embodiments, emitting moieties for chemiexcitation include quantum dots made of binary compounds such as cadmium selenide, lead selenide, cadmium sulfide, lead sulfide, indium phosphide, cadmium telluride, and indium arsenide. In some embodiments, quantum dots can also be made from ternary compounds such as cadmium selenide sulfide. Quantum dots can also be made free of heavy metals, such as CuInS/ZnS, and InP/ZnS.

In some embodiments, peroxyoxalate reagents for chemiexcitation are bis(2,4,6-trichlorophenyl) oxalate (TCPO), bis(2,4,5-trichlorophenyl)oxalate (2,4,5 TCPO), bis(1,1,1,3,3,3-hexafluoro-2-propyl)oxalate, bis[1-(1H)-2-pyridonyl]glyoxal, bis(2,4,5-trichlorophenyl-6-carbopentoxyphenyl)oxalate(CPPO),Bis(2,4-dinitro-6-methylphenyl)oxalate(DNNPO),bis(2,14-dinitrophenyl)oxalate(DNPO),bis(3-trifluoromehyi4-nitrophenyl)oxalate (TFMNPO), divanillyl oxalate.

In some embodiments, pyridonylglyoxal reagents for chemiexcitation are used such as Bis(1-[1H]-5-nitro-2-pyridonyl)glyoxal (NPG).

In some embodiments, oxalic anhydride reagents for chemiexcitation are used such as triphenylacetic oxalic anhydride (TPAOA), acetic oxalic anhydride (AOA).

In some embodiments, water soluble peroxyoxalate reagents for chemiexcitation are used such as peroxyoxamide:
2,2'-Oxalyl-bis[(trifluoromethanesulfonyl)imino]ethylene-bis(N-methylpyridinium)trifluoromethanesulfonate, and
2,2'-Oxalyl-bis[(trifluoromethanesulfonyl)imino]ethylene-bis(N-methylpyridinium)tetrafluoroborate

Modifications of chemiexcitation include the following:
Stimulating moieties dissolved in organic solvents such as diethyl phthalate, dimethoxyethane, dioxanes, DMSO, DMF, acetone, acetonitrile, ethyl acetate, tetrahydrofuran, triacetin, dodecane, siloxanes, olive oil, 3-Methoxy-3-Methyl-1-Butanol (MMB), PEG-400, glycerol and propylene carbonate, or in solvents containing surfactants, micelles, or reverse micelles.

In some embodiments, the reaction is enhanced by a catalyst such as sodium salicylate, tripropylamine, triethylamine, sodium acetate, tetrabutylammonium perchlorate, benzyltrimethylammonium hydroxide, oxalic, trichloroacetic, picric acid.

In some embodiments, cosolvent is used to promote miscibility. In some embodiments, cosolvent includes methanol, tert-butanol.

Emitting moieties attached to a particle also attached to a molecular recognition agent, or directly attached to a molecular recognition agent.

Freeing an intercalating dye from double-stranded DNA intercalation to allow it to be stimulated to emit, *e.g.,* as an assay for a nuclease used as a label on an antibody.

FRET from a dye excitable by the stimulating moieties to other dyes (or phosphors) which are not excitable in this way can allow proximity measurements and homogeneous assays without light sources or optical filters.

In some embodiments, a light signal is generated by contacting the emitter with an energy source other than electro-magnetic radiation, including chemically excited species or radiochemical emissions, which is capable of activating the emitter.

In some embodiments, the chemiluminescent reporters of the present disclosure may be coated or functionalized with water soluble moieties like poly(ethylene glycol) or hydrophilic polymers in order for reporters to become easily dispersible in water or aqueous solutions. In some embodiments, the chemiluminescent reporters may be coated or functionalized with moieties that reduce non-specific binding in analytical assays or tests.

In some embodiments, the chemiluminescent reporters of the present disclosure may be modified with molecular recognition elements, such as antibodies or aptamers. In some embodiments, the molecular recognition elements may be covalently attached to chemiluminescent reporters.

In some embodiments, the molecular recognition elements may be non-covalently attached to chemiluminescent reporters, such as by physical adsorption, receptor-ligand binding

In some embodiments, the present disclosure pertains to a method for the detection of at least one analyte within a sample. Such a method comprises the step of providing a chemiluminescent reporter. In some embodiments, the method further comprises contacting the chemiluminescent reporter to the sample. In some embodiments the method comprises the step of detecting the chemiluminescent signal of the chemiluminescent reporter. In some embodiments the method comprises the step of determining the presence of an analyte and quantifying the analyte based on the detected chemiluminescent signal.

In some embodiments, the present disclosure pertains to a method for the *in vitro* detection of at least one analyte within a sample. Such a method comprises the step of providing a chemiluminescent reporter. In some embodiments the method further comprises loading the chemiluminescent reporter into a porous material. In some embodiments, the method comprises contacting the sample with the porous material loaded with the chemiluminescent reporter. In some embodiments, the method comprises allowing the sample and the chemiluminescent reporter to flow through a porous membrane. In some embodiments, the method comprises detecting areas of chemiluminescent signal or absence of chemiluminescent signal on the membrane to indicate presence or absence of the at least one analyte.

In some embodiments, the present disclosure pertains to a method for *in vitro* detection of at least one analyte within a sample. Such a method comprises the steps of providing a chemiluminescent reporter; and providing at least one first molecular recognition element immobilized on a surface, where the immobilized molecular recognition element is capable of binding to the at least one analyte. In some embodiments, the method further comprises contacting the sample with the surface to allow binding of the at least one analyte to the molecular recognition element such that the analyte is immobilized. In some embodiments, the method comprises contacting the chemiluminescent reporter with the surface to allow binding of the chemiluminescent reporter to the at least one immobilized analyte; and measuring chemiluminescent signal from the chemiluminescent reporter to allow detection or quantification of the analyte.

In some embodiments, the surface comprises microfluidic chips, or paper microfluidics, or membranes, or microplates, or microbubbles for flotation, or transparent Surfaces. In some embodiments, the chemiluminescent reporter comprises at least one inorganic chemiluminescent particle; a shell encapsulating the at least one chemiluminescent particle: and a second molecular recognition moiety disposed on the shell. In some embodiments, the second molecular recognition moiety binds to the at least one immobilized analyte to generate the chemiluminescent t signal.

In some embodiments, the present disclosure relates to a magnetic chemiluminescent reporter. In some embodiments, the magnetic chemiluminescent reporter comprises at least one inorganic chemiluminescent particle. In some embodiments, the magnetic chemiluminescent reporter comprises a shell encapsulating the at least one inorganic chemiluminescent particle and at least one magnetic moiety disposed on the shell. In some embodiments, the magnetic chemiluminescent reporter comprises at least one molecular recognition moiety disposed on the shell. In some embodiments, the inorganic chemiluminescent particle forms the core of the chemiluminescent reporter.

In some embodiments, the magnetic chemiluminescent reporter comprises least one chemiluminescent particle and at least one magnetic particle or layer of magnetic material. In some embodiments, a shell encapsulates the at least one chemiluminescent particle and the at least on magnetic particle or layer of magnetic material. In some embodiments, the magnetic chemiluminescent reporter comprises at least one molecular recognition moiety disposed on the shell. In some embodiments, the inorganic chemiluminescent particle and the at least one magnetic particle or magnetic layer form the core of the chemiluminescent reporter.

In some embodiments, the present disclosure pertains to a method of detecting at least one analyte within a sample. Such a method comprises providing a magnetic chemiluminescent reporter. In some embodiments, the magnetic chemiluminescent reporter comprises at least one inorganic chemiluminescent particle and at least one magnetic particle or layer of magnetic material. In some embodiments, the magnetic chemiluminescent reporter further comprises a shell encapsulating the at least one inorganic chemiluminescent particle and the at least one magnetic particle or layer of magnetic material. In some embodiments, the reporter comprises at least one first molecular recognition moiety specific to the analyte disposed on the shell.

In some embodiments, the method further comprises contacting the aforementioned magnetic chemiluminescent reporter to the sample. In some embodiments, the method comprises concentrating the magnetic chemiluminescent reporter by use of a magnetic field. In some embodiments, the method comprises contacting the concentrated magnetic chemiluminescent reporter with a surface. In some embodiments, the surface is functionalized with at least one second molecular recognition moiety specific to the analyte.

In some embodiments, the method further comprises detecting a chemiluminescent signal of the magnetic chemiluminescent reporter; and determining the presence of an analyte or quantifying the analyte based on the detected chemiluminescent signal. In some embodiments, the first molecular recognition moiety binds to the at least one analyte to generate the chemiluminescent signal.

In additional embodiments, the present disclosure pertains to methods of detecting the chemiluminescent reporters in various settings, such as diagnostic settings. In some embodiments, the methods involve the detection of chemiluminescent signal from the chemiluminescent reporters. In some embodiments, chemiluminescent signal from a chemiluminescent reporter may be detected by excitation of the chemiluminescent reporter. In various embodiments, the chemiluminescent reporter may be excited by electrons, an electric field, photons, and combinations thereof. In some embodiments, chemiluminescent may be detected simultaneously with excitation using wavelength selective mechanisms. In some embodiments, the chemiluminescent signal may be enhanced by the application of heat.

In some embodiments, chemiluminescent signal is used to quantitatively or qualitatively obtain a signal in an assay by imaging with a film-based or digital camera (*e.g.,* a digital camera with a CMOS, CCD or other type of sensor). In some embodiments, chemiluminescent signal may be measured with a luminometer, fluorometer, spectrophotometer, or other similar instrument capable of measuring intensity of light.

In some embodiments, a cellphone, Smartphone, or portable electronic device such as, but not limited to, a tablet, personal digital assistant, or laptop can be used to detect chemiluminescence from the chemiluminescent reporters for qualitative or quantitative assay readout. In some embodiments, a cellphone or portable electronic device may be coupled to an attachment to allow chemiluminescent detection from chemiluminescent reporters to test for the presence or absence of an analyte in a sample.

In some embodiments, the present disclosure pertains to methods for enhancing the detection of chemiluminescent reporters. In some embodiments averaging techniques are used to achieve a higher signal-to-noise ratio of the chemiluminescent signal from chemiluminescent reporters used in an assay for detecting the presence or absence of an analyte.

In some embodiments, the chemiluminescent reporters of the present disclosure may be utilized in various assay settings. In some embodiments, the assay settings may include, without limitation, lateral flow, surface-bound assays, in flow through assays, assays associated with buoyant materials, or assays associated with magnetic materials for concentration or force stringency. In some embodiments, the present disclosure relates to compositions of matter comprising chemiluminescent reporters and porous membranes such as, but not limited to, nitrocellulose, glass fibers, and cotton fibers. In some embodiments, the compositions of matter comprising chemiluminescent reporters and porous membranes are used in assays for analyte detection.

In an embodiment, PCR is monitored by the emissions of fluors excited chemically, rather than by light. The fluors used can be intercalating dyes which become less bright as PCR produces more double-stranded product which sequesters them from chemiexcitation, or they can be de-quenched by nuclease action as in Taqman assays. Amplification can be by PCR, but also by other methods such as RPA (*e.g.*, with hydrolysis probe). NASBA, LAMP, or HDA.

In an embodiment, protease activity is reported by emissions of chemically excited fluors after proteolysis of a peptide or protein associated with a fluor and a quencher or a quenching material or surface.

In an embodiment, a nuclease, protease, carbohydrase or oxidoreductase associated with a molecular recognition element is used to detect the target of the molecular recognition element. The enzyme reports the presence of the target by changes in the emissions of fluors whose chemical environment is changed by the action of the enzyme.

In an embodiment, an enzyme-labeled immunoassay is performed using a detector antibody conjugated to a nuclease, such as benzonase. A signal is obtained by contacting the detector antibody with a solution containing nucleic acids with associated intercalating fluorescent dyes, and chemiexcitation reagents.

In an embodiment, an immunoassay is conducted by transfer of energy from a fluor which is chemically excited to one which is not chemically excited in the same environment, by coupling each of them onto an antibody, where the two antibodies can form a sandwich pair with a target. In the presence of the target they are close enough together for energy transfer to occur and the acceptor fluorescence is observed. This approach can serve as the basis of a mix and read homogeneous immunoassays.

Protein single oxygen generators disclosed but not claimed, like miniSOG coupled to antibodies can replace the oxygen generating particles in the AlphaLisa system.

An embodiment disclosed but not claimed, is the performance of lateral or vertical flow assays using fluorescent reporters (*e.g.*, silica, inorganic, latex, polymeric, acrylic, gold or carbohydrate particles, QDots, antibody conjugates, phage, etc.) excitable by singlet oxygen or chemi-excitation as reporter particles, along with membranes or filters composed of nitrocellulose, silica, or other wettable and permeable materials.

Chemical excitation of multiple fluors can support parallel or multiplex analytical or diagnostic tests with reduced equipment complexity and/or easier access to a variety of reporters.

In an embodiment, emission by chemical is partially captured by persistent luminescence materials to increase detectability by maximizing the number of photons delivered to the eye or a chemical or electronic detector.

In an embodiment, materials of a container also serve to absorb light of wavelengths not most useful in determining the concentration of analyte, while selectively transmitting light of wavelengths more useful in determining the concentration of analyte.

Emission can be observed by eye, by smartphone camera, by cMOS, APD, CCD, PMT, film, or photodiode, or with intensification by an IR viewer, microchannel device or image intensifier.

In an embodiment, the presence of analyte triggers location or relocation of an imaging moiety in a location where its detectability is either enhanced or reduced. For example, closer to or farther away from a detector in the presence of a dye which captures emitted light, or in the presence of a competing moiety which suppresses energy transfer or chemiexcitation or illumination.

### II. Analytes of Interest and Source

The methods and compositions disclosed herein may be utilized to detect various analytes of interest from various specimens. For instance, in some embodiments, analytes of interest include, without limitation, nucleic acids such as genomic DNA, methylated DNA, specific methylated DNA sequences, messenger RNA, fragmented DNA, chromosomal DNA, mitochondrial DNA, fetal DNA, fetal RNA, rDNA, cDNA, fragmented RNA, fragmented mRNA, rRNA, viral RNA, siRNA, microRNA, SSU RNAs, LSU-rRNAs, 5S rRNA, spacer region DNA from rRNA gene clusters, 5.8S rRNA, 4.5S rRNA, 10S RNA, RNAseP RNA, guide RNA, telomerase RNA, snRNAs -e.g. U1 RNA, scRNAs, polymerase chain reaction (PCR) products, cpDNA, artificial RNA, plasmid DNA, oligonucleotides, polyA mRNA, RNA/DNA hybrid, pathogen DNA, pathogen RNA, replication protein A (RPA) amplification product, loop-mediated isothermal amplification product (LAMP), restriction fragments, YAC, BAC, cosmid, ,metabolite, metabolic intermediate, hormone (such as insulin, testosterone, and HCG), organelle, biomarker, lipid, carbohydrate, pathogen carbohydrate or protein, human protein, glycoprotein, lipoprotein, phosphoprotein, specific phosphorylated or acetylated variant of a protein, or viral coat proteins, cell surface receptor, peptides, drugs, spores, enzyme substrate, enzyme, and enzyme reaction product, , anthrax spore, teichoic acid, prion, chemical toxins (such as pesticides, atrazine, and digoxin), and other chemical warfare or defense agents, biological warfare agent, or pollutant, invasive plant or animal species, Pesticides and herbicides, Residues remaining after cleaning of equipment or surfaces, bedbug proteins, Bedbugs, characteristic proteins of genetically-modified plants or animals, toxins, pathogens.

Analytes to be detected or quantified may be isolated from various sources. For instance, in some embodiments, analytes may be isolated from cells, tissues, or body fluids, such as a biopsy specimen, blood, serum, plasma, stool, saliva, sweat, sputum, vomit, CSF, lavage fluid, tears, ocular fluids, transcellular fluid, urethral or genital secretions, exudate from lesions or areas of inflammation, nasal wash, nasal swab, throat swab, urine, hair, cell lysate, circulating tumor cells, FNAB cells, FACS fraction, immunomagnetic isolate, air filtrate, FFPE slices, fresh-frozen specimens, fresh tissue, frozen tissue, neutral formalin-treated tissue, a formalin fixed paraffin embedded tissue block, an ethanol-fixed paraffin-embedded tissue block, surgical site, FACS-sorted population, laser-capture microdissection fraction, magnetic separation subpopulation, FFPE extracts. In some embodiments, analytes may be obtained from environmental samples from the soil, air, or water, agricultural products (grains, seeds, plants, meat, livestock, vegetables, rumen contents, milk, *etc*.); contaminated liquids; surface scrapings or swabbings; biofilms, cell cultures, pharmaceutical production cultures, CHO cell cultures, bacterial cultures, virus-infected cultures, microbial colonies, and combinations thereof. In some embodiments, an entire sample source may be analyzed. In some embodiments, only a portion of a sample source may be analyzed.

In some embodiments, the analyte to be detected may be obtained from surfaces or components of clothing, shoes, garments, personal protective gear and personal equipment, or other gear. bathrooms, military settings, equipment or objects in the vicinity of or near a facility for the production of agricultural or food products. In some embodiments the analyte to be detected may be obtained from any surfaces or components of objects suspected of contamination with illicit substances or hazardous materials or analytes associated with the production of illicit substances or hazardous materials.

### III. Sample Preparation

In various embodiments, the assays can include one or more sample-preparation steps. In some embodiments, the sample preparation steps may utilize various sample preparation agents. In some embodiments, sample preparation may include, without limitation, concentrating, enriching, and/or partially purifying the analytes of interest. For instance, in some embodiments, the samples may be pre-treated by centrifugation, sedimentation, fractionation, field-flow fractionation, elutriation, monolithic separation, extraction, adsorption, protease, nuclease, dialysis, osmosis, buffer exchange, partitioning, washing, dewaxing, leaching, lysis, osmolysis, amplification, denature/renaturation, crystallization, freezing, thawing, cooling/heating, degasification, sonication, pressurization, drying, magnetophoresis, electrophoresis, dielectrophoresis, acoustophoresis, precipitation, microencapsulation, sterilization, autoclaving, germination, culturing, PCR, disintegration of tissue, extraction from FFPE, LAMP, NASBA, emulsion PCR, phenol extraction, silica adsorption, immobilized metal affinity chromatography (IMAC), filtration, affinity capture, capture from a large volume of a dilute liquid source, air filtration, surgical biopsy, FNA, flow cytometry, laser capture microdissection, and combinations thereof.

In some embodiments, sample preparation may include, without limitation, use of various concentrations of a dilute species from a liquid or gaseous environment using a filter, isolation of a subset of cells from a complex blood sample, breakage of cells to liberate analytes of interest, extraction of the analyte from a solid sample, or removal of lipids and particulates, which could interfere with later analysis.

In some embodiments, sample preparation may involve amplification of the analyte to be detected. For instance, amplification may include the use of the polymerase chain reaction to amplify nucleic acids or nucleation chain reaction to amplify prion proteins, or growth of an organism. Another way to amplify the detectability of an analyte is to grow an assembly of biomolecules, such as an actin filament or immune complex. Another method is to use a nucleating agent (*e.g*., of bubbles, crystals or polymerization) as an element of the analyte. Where available, these methods can greatly facilitate subsequent analysis.

The analytes of the present disclosure can be modified in various manners. In some embodiments, the analytes can be modified by labeling, conjugation, methylation, esterification, dephosphorylation, phosphorylation, acetylation, deacetylation, methylation, demethylation, denaturation, oxidation, conjugation, haloacetic acid modification, hatching, growth, excystation, passaging, culture, de-blocking, proteolysis, nuclease digestion, cDNA preparation, amplification, DNA ball preparation, PEGylation, clonal amplification, multiplication, charge enhancement, hybridization, antibody binding, adsorption, aptamer binding, photo-linking, reduction, oxidation, and combinations thereof.

### IV. Assay Reagents

In some embodiments, the analytes of the present disclosure may be detected by assays using various fluorescers. The fluorescers can also be attached to the molecular recognition elements which can be part of, all of, associated with, or attached to labels include a nanoparticle, gold particle, silver particle, silver, copper, zinc, iron, iron oxide, or other metal coating or deposit, polymer, drag tag, magnetic particle, buoyant particle, microbubble, metal particle, charged moiety, silicon dioxide, with and without impurities (*e.g.,* quartz, glass, *etc*.), poly(methylmethacrylate), polyimide, silicon nitride, gold, silver, quantum dot, CdS, carbon dot, a phosphor such as silver-activated zinc sulfide or doped strontium aluminate, a fluor, a quencher, polymer, PMMA, polystyrene, retroreflector, bar-coded or labeled particle, porous particle, pellicular particle, solid particle, nanoshells, nanorods, IR absorbers, microwave absorbers, microspheres, liposomes, microspheres, polymerization initiators, photografting reagents, proteins, molecular recognition elements, linkers, self-assembled monolayers, PEG, dendrimers, charge modifiers, PEG, silane coupling agents, initiators of growth from polymer grafts from the label surface, stabilizing coatings, zwitterions, zwitterionic peptides, zwitterionic polymers, magnetic materials, magnetic materials of Curie temperature below 200°C, enzyme. microbial nanowire..." DNA including aptamer sequences, phage modified for conductivity, fusions or conjugates of detectable elements with molecular recognition elements, Streptavidin, NeutrAvidin, Avidin, or biotin-binding proteins, biotin, biotinylated molecules, biotinylated polymers, biotinylated proteins, anti-antibody aptamer, aptamer directed to antibody-binding protein, an azide or terminal alkyne or other click chemistry participant, and combinations thereof. In some embodiments, the surface of a reporter is modified by covalent attachment of molecular recognition elements. In some embodiments, the surface of a reporter is modified by adsorption of molecules for colloidal stability or molecular recognition.

In some embodiments, assay elements may be functionalized with various functional groups on their surfaces. In some embodiments, components of the assay may be coated or functionalized with moieties that reduce non-specific binding in analytical assays or tests. Exemplary functional groups include, without limitation, amine groups, carboxyl groups, aldehydes, ketones, hydroxyls, maleimide groups, sulfhydryl groups, thiols, hydrazides, anhydrides, alkenes, alkynes, azides, and combinations thereof. In other embodiments, assay elements can be coupled to aldehydes on antibodies created by oxidizing the polysaccharides on the F_{c} portion of the antibody with periodate. In further embodiments, Protein A or other proteins that bind specifically to the F_{c} portion of an antibody can be attached to an assay element, and then used to bind to an antibody in an oriented manner.

In some embodiments, the assay elements of the present disclosure may be utilized in various assay settings. In some embodiments, the assay settings may include, thermal cyclers, incubators, electrochemical cells, lateral flow media, microtiter wells, surface-bound assays, flow through assays, assays associated with buoyant materials, relocation assays, nanopore..." plasmonic layers with holes below 120 nm diameter for extraordinary optical transmission, microchannels, microdroplets, nanowells, or assays associated with magnetic materials for concentration or force stringency. In some embodiments, the present disclosure relates to compositions of matter comprising assay elements and porous membranes such as, but not limited to, nitrocellulose, glass fibers, and cotton fibers.

### V. Molecular Recognition Element (MRE)

Many analytical methods, including those of interest in the present invention, involve molecular recognition, and also transduction of the molecular recognition event into a usable signal. Molecular recognition refers to the high affinity and specific tendency of particular chemical species to associate with one another, or with organisms or viruses displaying target chemical species. Well-known examples of molecular recognition include the hybridization of complementary DNA sequences into the famous double helix structure with very high affinity, and the recognition of foreign organisms or molecules in the bloodstream by the antibodies produced by mammals, or selected analytes by deliberately selected monoclonal antibodies. There are many other examples of molecular recognition elements, including the recognition of carbohydrate molecules by lectins, nucleic acid recognition by proteins and nucleic acid analogs, the binding of analytes by antibody fragments, derivatives, and analogs, and a host of other examples.

In some embodiments, the label elements of the present disclosure may also be associated with various molecular recognition elements. In some embodiments, the molecular recognition elements may be part of, associated with, or attached to labels. In some embodiments, molecular recognition elements can include, without limitation, antibody, antibody fragment, antibody analog, affibody, camelid or shark antibody analog, nucleic acid, carbohydrate, aptamer, ligand, chelators, peptide nucleic acid, locked nucleic acid, backbonemodified nucleic acid, DARPin, molecularly imprinted polymers, lectin, padlock probe, substrate, receptor, viral protein, mixed, cDNA, metal chelate, boronate, peptide, enzyme substrate, enzyme reaction product, lipid bilayer, cell, tissue, microorganism, yeast, bacterium, parasite, protozoan, virus, antigen, hapten, biotin, hormone, drug, anti-RNA/DNA hybrid antibody, mutS, anti-DNA antibody, anti-methylation antibody, or an anti-phosphorylation antibody.

### VI. Covalent Modification Agents

In some embodiments, the assay involves a covalent modification agent, which may be a catalytic or reactive moiety. In some embodiments, the covalent modification agent functions as a template generator element. The template generator element can be a protein, such a nucleic acid-modifying enzyme. These enzymes include without limitation such as a ligase, helicases, methylase, kinase, demethylase, dephosphorylase, phosphatase, RNase H, polymerase, exonuclease or endonuclease. The covalent modification agent may include biotin ligase, sumoylation enzymes, sortases, or ubiquitin ligases, lipoyl protein ligase, or lipoic acid ligase. Covalent modification agents may be associated with, expressed in, coupled to, or displayed on particles, surfaces, cells, or phages. Chemical covalent modification agents include click reagents, aldehydes, cross-linkers, supported metals such as gold and platinum, and photoreactive moieties.

### VII. Amplification or Signal Enhancement Methods

An assay includes a molecular recognition event that is detected by a usable signal. In certain embodiments, the signal may be amplified or enhanced by a signal enhancement method, which may act upon the analyte, molecular recognition element, a label associated to either one of them, or a component of such label.

Many applications discussed herein mention assays in which detection of analyte involves direct binding of the reporter to the analyte at or in a specific region of interest and an increase in signal from the region of interest indicates a positive signal. In another embodiment, a readout method by which the analyte is detected is by determination of the presence or absence of the label in locations different from the locations expected in the absence of the analyte. Of particular interest are assays in which binding (or the suppression of binding, or competition) gives rise to the presence or absence of a signal. For instance, a phosphorescent label can be displaced from a pre-specified region by the presence of analyte, and then a decrease in luminescence from that region or an increase in luminescence elsewhere would indicate a positive signal. In some embodiments, luminescence is used to quantitatively or qualitatively obtain a signal in an assay by imaging with a film-based or digital camera (*e.g.,* a digital camera with a CMOS, CCD or other type of sensor). In some embodiments, luminescence may be measured with a luminometer, fluorometer. PMT, avalanche photodiode, spectrophotometer, or other similar instrument capable of measuring intensity of light.

In some embodiments, the signal amplification or enhancement method may include hatching, growth, PCR, solid-phase PCR, RPA, LATE, EATL, RCA, LAMP, 3SR, LCR, SDA, MDA, HDA, or hot-restart amplification, solid-phase RCA, silver staining, metal deposition or plating, nickel, copper or zinc deposition, gold particle growth, polymerization, particle binding, grafting, photografting, click chemistry, a copper(I)-catalyzced 1,2,3-triazole forming reaction between an azide and a terminal alkyne, and combinations thereof.

In some embodiments, the molecular recognition elements include protein beacons, analyte-sensitive fluorescent proteins, allosteric molecular beacons, hairpin peptide beacons, molecular aptamer beacons, quantum dot aptamer beacons, molecular beacon aptamers, allosteric fluorescent protein biosensors, or molecular switch sensors.

In some embodiments, a luminescence signal from a reporter such as a chemiluminescence-active enzyme, fluor, or phosphor may be read or detected by an optical sensor such as, but not limited to, a charge-coupled device (CCD) sensor, CCD image sensor, complementary metal-oxide-semiconductor (CMOS) sensor, CMOS image sensor, camera, cell phone camera, photodiode, avalanche photodiode, single-photon avalanche diode, superconducting nanowire single-photon detector, photoresistor, photomultiplier, photomultiplier tube (PMT), phototube, photoemissive cell, photoswitch, phototransistor, photonic crystal, fiber optic sensor, electro-optical sensor, luminometer, or fluorometer. In some embodiments the luminescence signal from the label elements may be read, detected, or inferred from photochemical reactions, such as those that occur in photographic film, with the photochemical reactions stimulated or initiated by luminescence from the label elements. In some embodiments the luminescence signal from the label elements may be read or detected visually by the naked eye, or with the assistance of an optical amplifier or intensifier.

In some embodiments, a cell phone, smart phone, or portable electronic device such as, but not limited to, a tablet, personal digital assistant, or laptop can be used to detect signals from reporters for qualitative or quantitative assay readout. In some embodiments, a cell phone or portable electronic device may be coupled to an attachment device to detect or test for the presence or absence of an analyte in a sample. In some embodiments, averaging techniques are used to achieve a higher signal-to-noise ratio of detection method in an assay for detecting the presence or absence of an analyte.

### VIII. Specificity Enhancement

In some embodiments, the specificity of detection of analytes may be enhanced through removal of non-specifically bound labels by chemical or physical means. In some embodiments, chemical means of removal include denaturants, temperature, acids, bases, osmolytes, surfactants, polymers, and solvents. In some embodiments, physical means of removal include force, vibration, buoyancy, washing, centrifugation, sedimentation field-flow, magnetic force, electrophoretic force, dielectrophoretic force, sonication, and lateral force. In some embodiments, susceptibility to means of removal may be enhanced by incorporation of moieties particularly responsive to means of removal, such as charged or dense moieties for electrophoretic or sedimentation-based removal.

### IX. Location of Analysis

Various locations may be used for sample analysis. In some embodiments, the location of the steps of the analysis, which may be used singly or in combination, include microtiter plates, tubes, the surfaces of particles or beads, nanowell arrays, flow injection analysis apparatus, microfluidic chips, conductive surfaces, temperature-controlled environments, pressure chambers, ovens, irradiation chambers, electrophoretic, field-flow and chromatographic apparatus, microscope stages, luminometers, Coulter principle devices, cantilever and FET sensors, vacuum chambers, electron optical apparatus, single-molecule detection apparatus, single-molecule fluorescence detection apparatus, surfaces bearing nanoholes, electrodes or pillars, emulsions, lateral flow membranes, flow through membranes, lateral flow assay readers, flow through assay readers, gel documentation systems, robotic apparatus, and combinations thereof. Rotation and flow devices, or fast electronic or mechanical shutters, enhance sensitivity by allowing detection of luminescence before it reduces with time. Flow injection analysis, "Lab on a chip" and "Lab on a CD" approaches can be desirable in some embodiments. In some embodiments, it may be advantageous to perform more than one type of analysis in series, either fractionating a sample using or based on the results of one method before performing an additional method, or by interpreting together the results of multiple methods.

In an embodiment, assays may be used to detect specific sequences of nucleic acids. Nucleic acids may be detected from a variety of sample types depending on the application, such as tumor cell lysates for cancer diagnostics. The analyte nucleic acid is recognized by another nucleic acid that functions as MRE. The MRE nucleic acid is physically associated to a CMS such as ligase or a helicase. Assay components are added such that the ligase or helicase generate templates that are specifically associated to the molecular recognition of the MRE nucleic acid to the analyte nucleic acid. These templates are then subject to an amplification step by polymerase chain reaction (PCR), isothermal PCR, loop-mediated isothermal amplification (LAMP), or replication protein A (RPA) amplification, and this amplified signal is detected by appropriate means. Assay elements may be functionalized with single stranded DNA or RNA that hybridizes with part of a complementary strand from a sample that is specific to an analyte. In some embodiments the target complementary nucleic acid strand has a specific tag which is introduced during amplification or by other means and allows the tagged strand to be captured by a surface that recognizes the tag. For example, an amplification product may be biotinylated so that it binds to surfaces coated with avidin or is retained at particular locations in a lateral-flow strip. In other embodiments, the surface is functionalized with a nucleic acid strand that specifically hybridizes with a small segment of the target nucleic acid strand, which is long enough to also allow hybridization with a reporter containing a complementary sequence to a different part of the target nucleic acid strand. Multiplexed lateral flow assays can be readily designed by adding multiple test lines to a strip and using reporters functionalized with different molecular recognition elements to bind specifically at each test line.

### X. Detector Devices

The luminescent signal output is detected by light detector such as, but not limited to, charged coupled device (CCD), avalanche diode, (multi-pixel photon counter) MPPC or silicon photomultiplier (SiPMT), complementary metal-oxide-semiconductor (CMOS) sensor, scientific CMOS (sCMOS) sensor, photomultiplier tubes (PMT), photodiode, camera, cellphone camera, web camera, smart watch camera, or any light detector. The light detector can be function as a point-of-care device connected and controlled via wired or wireless connection by personal computer, laptop, tablet, smart phone, smart watch, or any similar devices with computing and displaying capabilities. The wireless connection includes, but not limit to, Bluetooth, Wi-Fi, NFC.

The assay can be done in plate format and signals are readout by plate reader equipped with photomultiplier tubes (PMT), avalanche diode, (multi-pixel photon counter) MPPC or silicon photomultiplier (SiPMT), charged coupled device (CCD) sensor, complementary metal-oxide-semiconductor (CMOS) sensor, scientific CMOS (sCMOS) sensor.

### XI. Assay Formats (The claimed invention relates to a lateral or vertical flow assay. Other assay formats, e.g. ELISAs, are disclosed but not claimed.)

### A. ELISAs

Immunoassays, in their most simple and direct sense, are binding assays. Certain preferred immunoassays are the various types of enzyme linked immunosorbent assays (ELISAs) and radioimmunoassays (RIA) known in the art. Immunohistochemical detection using tissue sections is also particularly useful. However, it will be readily appreciated that detection is not limited to such techniques, and western blotting, dot blotting, FACS analyses, and the like may also be used.

In one exemplary ELISA, the antibodies of the disclosure are immobilized onto a selected surface exhibiting protein affinity, such as a well in a polystyrene microtiter plate. Then, a test composition suspected of containing the antigen is added to the wells. After binding and washing to remove non-specifically bound immune complexes, the bound antigen may be detected. Detection may be achieved by the addition of another antibody that is linked to a detectable label. This type of ELISA is a simple "sandwich ELISA." Detection may also be achieved by the addition of a second antibody, followed by the addition of a third antibody that has binding affinity for the second antibody, with the third antibody being linked to a detectable label.

In another exemplary ELISA, the samples suspected of containing the antigen are immobilized onto the well surface and then contacted with the antibodies of the disclosure. After binding and washing to remove non-specifically bound immune complexes, the bound antibodies are detected. Where the initial antibodies are linked to a detectable label, the immune complexes may be detected directly. Again, the immune complexes may be detected using a second antibody that has binding affinity for the first antibody, with the second antibody being linked to a detectable label.

Irrespective of the format employed, ELISAs have certain features in common, such as coating, incubating and binding, washing to remove non-specifically bound species, and detecting the bound immune complexes. These are described below.

In coating a plate with either antigen or antibody, one will generally incubate the wells of the plate with a solution of the antigen or antibody, either overnight or for a specified period of hours. The wells of the plate will then be washed to remove incompletely adsorbed material. Any remaining available surfaces of the wells are then "coated" with a nonspecific protein that is antigenically neutral with regard to the test antisera. These include bovine serum albumin (BSA), casein or solutions of milk powder. The coating allows for blocking of nonspecific adsorption sites on the immobilizing surface and thus reduces the background caused by nonspecific binding of antisera onto the surface.

In ELISAs, it is probably more customary to use a secondary or tertiary detection means rather than a direct procedure. Thus, after binding of a protein or antibody to the well, coating with a non-reactive material to reduce background, and washing to remove unbound material, the immobilizing surface is contacted with the biological sample to be tested under conditions effective to allow immune complex (antigen/antibody) formation. Detection of the immune complex then requires a labeled secondary binding ligand or antibody, and a secondary binding ligand or antibody in conjunction with a labeled tertiary antibody or a third binding ligand.

"Under conditions effective to allow immune complex (antigen/antibody) formation" means that the conditions preferably include diluting the antigens and/or antibodies with solutions such as BSA, bovine gamma globulin (BGG) or phosphate buffered saline (PBS)/Tween. These added agents also tend to assist in the reduction of nonspecific background.

The "suitable" conditions also mean that the incubation is at a temperature or for a period of time sufficient to allow effective binding. Incubation steps are typically from about 1 to 2 to 4 hours or so, at temperatures preferably on the order of 25°C to 27°C, or may be overnight at about 4°C or so.

Following all incubation steps in an ELISA, the contacted surface is washed so as to remove non-complexed material. A preferred washing procedure includes washing with a solution such as PBS/Tween, or borate buffer. Following the formation of specific immune complexes between the test sample and the originally bound material, and subsequent washing, the occurrence of even minute amounts of immune complexes may be determined.

To provide a detecting means, the second or third antibody will have an associated label to allow detection. Preferably, this will be an enzyme that will generate color development upon incubating with an appropriate chromogenic substrate. Thus, for example, one will desire to contact or incubate the first and second immune complex with a urease, glucose oxidase, alkaline phosphatase or hydrogen peroxidase-conjugated antibody for a period of time and under conditions that favor the development of further immune complex formation (*e.g.,* incubation for 2 hours at room temperature in a PBS-containing solution such as PBS-Tween).

After incubation with the labeled antibody, and subsequent to washing to remove unbound material, the amount of label is quantified, *e.g.,* by incubation with a chromogenic substrate such as urea, or bromocresol purple, or 2,2'-azino-di-(3-ethyl-benzthiazoline-6-sulfonic acid (ABTS), or H₂O₂, in the case of peroxidase as the enzyme label. Quantification is then achieved by measuring the degree of color generated, *e.g.,* using a visible spectra spectrophotometer.

In another embodiment, the present disclosure contemplates the use of competitive formats. This is particularly useful in the detection of antibodies in sample. In competitionbased assays, an unknown amount of analyte or antibody is determined by its ability to displace a known amount of labeled antibody or analyte. Thus, the quantifiable loss of a signal is an indication of the amount of unknown antibody or analyte in a sample.

Here, the inventor proposes the use of labeled monoclonal antibodies to determine the amount of target antibodies in a sample. The basic format would include contacting a known amount of monoclonal antibody (linked to a detectable label) with antigen. The antigen preferably attached to a support. After binding of the labeled monoclonal antibody to the support, the sample is added and incubated under conditions permitting any unlabeled antibody in the sample to compete with, and hence displace, the labeled monoclonal antibody. By measuring either the lost label or the label remaining (and subtracting that from the original amount of bound label), one can determine how much non-labeled antibody is bound to the support, and thus how much antibody was present in the sample.

### B. Lateral Flow Assays

Lateral flow assays, also known as lateral flow immunochromatographic assays, are simple devices intended to detect the presence (or absence) of a target analyte in sample (matrix) without the need for specialized and costly equipment, though many laboratory-based applications exist that are supported by reading equipment. Typically, these tests are used as low resources medical diagnostics, either for home testing, point of care testing, or laboratory use. A widely spread and well-known application is the home pregnancy test.

The technology is based on a series of capillary beds, such as pieces of porous paper, glass fibers, microstructured bed, or sintered polymer. Each of these elements has the capacity to transport fluid (*e.g.,* urine) spontaneously. The first element (the sample pad) acts as a sponge and holds an excess of sample fluid. Once soaked, the fluid migrates to the second element (conjugate pad) in which the manufacturer has stored the so-called conjugate, a dried format of bio-active particles (see below) in a salt-sugar matrix that contains everything to guarantee an optimized chemical reaction between the target molecule (*e.g.,* an antigen) and its chemical partner (*e.g*., antibody) that has been immobilized on the particle's surface. While the sample fluid dissolves the salt-sugar matrix, it also dissolves the particles and in one combined transport action the sample and conjugate mix while flowing through the porous structure. In this way, the analyte binds to the particles while migrating further through the third capillary bed. This material has one or more areas (often called stripes) where a third molecule has been immobilized by the manufacturer. By the time the sample-conjugate mix reaches these strips, analyte has been bound on the particle and the third 'capture' molecule binds the complex. After a while, when more and more fluid has passed the stripes, particles accumulate and the stripearea changes color. Typically, there are at least two stripes: one (the control) that captures any particle and thereby shows that reaction conditions and technology worked fine, the second contains a specific capture molecule and only captures those particles onto which an analyte molecule has been immobilized. After passing these reaction zones, the fluid enters the final porous material - the wick - that simply acts as a waste container. Lateral Flow Tests can operate as either competitive or sandwich assays. Lateral flow assays are disclosed in U.S. Patent 6,485,982.

### XII. EXAMPLES

The following examples are included to demonstrate preferred embodiments. It should be appreciated by those of skill in the art that the techniques disclosed in the examples that follow represent techniques discovered by the inventor to function well in the practice of embodiments, and thus can be considered to constitute preferred modes for its practice.

### Example 1: Singlet oxygen excitation of LFA reporter particles

In an exemplary embodiment, a line of monoclonal antibody recognizing the NS 1 protein of serotype 2 of dengue virus is deposited across a nitrocellulose lateral flow membrane. 50 microliters of human fingerstick blood is applied to the membrane through a Vivid blood filter. This is followed by 100 microliters of buffer containing nanoparticles comprising thioxene and a europium chelate and decorated with a second monoclonal antibody to the dengue NS1 protein, where the first and second antibodies are capable of binding simultaneously to the same NS1 protein molecule. The lateral flow membrane is then washed with an additional 50 microliters of buffer and three drops of a solution containing the singlet oxygen generator protein is applied. The lateral flow membrane is then illuminated for one minute, then imaged for light emission in a closed chamber for 10 seconds, and the presence of a brightly emitting line at the location of the line of capture antibodies is taken as evidence of infection by dengue virus.

### Example 2: Chemiexcitation of LFA reporter particles for sensitive imaging detection

A lateral flow sandwich immunoassay is conducted on a porous membrane such as Fusion 5 strip with a sandwich pair of antibodies recognizing HIV core antigen p24. The reporter particles bearing the detector antibody are fluorescent. A human plasma sample is mixed with a biotinylated antibody recognizing an epitope of HIV p24 and with fluorescent nanoparticles bearing a different antibody recognizing a second epitope of p24. The mixture is flowed along a Fusion 5 LFA membrane with a neutravidin test line and anti-species control line. After the sample has been run, 200 microliters of a solution of oxalate ester and hydrogen peroxide is applied to the strip, which is then imaged with a cell phone camera in a dark box. The chemiexcitation of the fluors on the particle produces a bright emission at the test line, which is numerically integrated and divided by the emission at the control line to estimate the concentration of p24 in the original sample.

In a modification, the temperature during the signal development step with solution of oxalate ester and hydrogen peroxide is heated up and held above ambient temperature to speed up the rate of chemiluminescent reaction. The enhanced light signal is then detected with a cell phone camera in a dark box.

### Example 3: Chemiluminescence resonance energy transfer (CRET)

Homogeneous assay by FRET between chemiexcited donor and non-chemiexcitable acceptor 200 nanometer fluorescent particles with a fluorescence emission maximum near 490 nanometers are modified with antibodies to capsular polysaccharide of *Burkholderia pseudomallei.* Other antibodies recognizing the same target are modified with Alexa Fluor 488 dye. The particles and Alexa Fluor 488 dye-conjugated antibodies are freeze-dried in an optical cuvette. For use, 500 microliters of a human urine sample are added to the cuvette, followed by a diethyl phthalate solution of oxalate ester and 10 microliters of hydrogen peroxide solution. After mixing, light emission near the Alexa Fluor 488 dye emission maximum of 525 nanometers is ascribed to energy transfer between the chemiexcited fluors of the fluorescent particles and Alexa Fluor 488 dye on the detector antibody. Alexa Fluor 488 dye is not chemiexcited under these conditions, so its excitation and emission occur only in the presence of bridging target analyte polysaccharide. Alexa Fluor 488 dye emission is therefore interpreted as evidence of infection of the urine donor by *Burkholderia pseudomallei.*

In a modification of the above example, the FRET donor and acceptor are quantum dots. In which the donor quantum dots are excitable by chemiluminescent substrate while the acceptor quantum dots are selected for non-chemiexcitable.

In another modification, the FRET donors are quantum dots and acceptor are non-chemiexcitable fluorescent dye.

### Example 4: Chemiexcitation of quantum dot particles for sensitive detection in lateral-flow format

In one example, quantum dots conjugated to antibody can be used as a reporter in lateral-flow assay. A sample is contacted quantum dot such as CdSe or ZnO, particles bearing antibodies to a protein of diagnostic interest and applied to the upstream end of a dry lateral-flow wicking matrix. In the preferred embodiment, the membrane is constructed from nitrocellulose on a polymer-based backing support material. The membrane is spotted with a test line for analyte detection comprising antibodies that are either polyclonal or monoclonal and bind to a different epitope of the target protein than the antibodies on the quantum dot particles. The membrane is also spotted with a control line which may consist of some type of molecular recognition moiety that binds specifically to the quantum dot particles. An absorbent pad is selected to effectively wick the liquid sample through the strip, and ideally prevent backflow, which can hamper sensitivity. A second antibody which recognizes the protein is positioned downstream on the lateral-flow wicking matrix. After development, chemiluminescent substrate containing freshly prepared mixture of hydrogen peroxide and peroxyoxalate is added to the lateral-flow material. The lateral-flow strip is immediately imaged or scanned for the presence of light signal at the positions of test and control lines using the camera or light detector.

In a modification, different antibodies are conjugated quantum dots with different emission colors separately to detect multiple targets in the same sample. The capture antibodies for each target can be in a single line or in multiple separate lines. After development, chemiluminescent substrate containing freshly prepared mixture of hydrogen peroxide and peroxyoxalate is added to the lateral-flow material. The lateral-flow strip is immediately imaged or scanned for the presence of light signal at the test and control lines using the color camera or light detector with proper filters to separate the signals for each target.

### Example 5: Aptamer with an intercalating fluorescent dye as reporter

A DNA aptamer recognizing VEGF protein is contacted with an intercalating fluorescent dye. The dye-loaded aptamer is placed in an oxalate-based chemiexcitation solution, and a sample of human blood is added. Binding of the aptamer to the VEGF protein causes conformational changes that liberate the fluorescent dye into solution. Liberated dye is chemically excited, and the resulting fluorescence emission is used to estimate the concentration of VEGF protein in the blood sample.

### Example 6: Detection and quantification of double stranded nucleic acids with an intercalating fluorescent dye as reporter

In an exemplary embodiment, a sample containing target DNA is added to polymerase chain reaction (PCR) master mix containing intercalating fluorescer and a primer pair that is specific to the target DNA. Optionally, the PCR master mix also contains a non-intercalating fluorescer such as ROX reference dye. The reaction mix is subjected to temperature cycling from 25 to 45 cycles of annealing and denaturation to amplify the target DNA. After amplification, the amplified DNA is quantified by adding chemiluminescent substrate containing a freshly-prepared mixture of hydrogen peroxide and peroxyoxalate. The sample is immediately imaged or scanned for the presence of light signal using a camera or light detector. The signal of the intercalating fluorescer is inversely related to the amount of amplified DNA. If a non-intercalating reference dye is used, the ratio of intercalating dye to the non-intercalating can be used to calculate the amount of amplified PCR. Melting temperature of the amplified DNA can be derived by gradually changing the temperature from annealing to denaturation, or vice versa, while monitoring the amount of chemiluminescent signal. A sharp change in the chemiluminescent signal indicates the melting temperature of the amplified DNA.

In a modification, the DNA amplification reaction is an isothermal reaction such as recombinase polymerase amplification (RPA), loop mediated isothermal amplification (LAMP), or other isothermal amplification technique.

### Example 7: Detection and quantification of analyte in plate format

In another example, target analyte can be detected and measured in a chemiluminescent immunosorbent assay. The sample well is coated with antibody that recognizes the target analyte and blocked with BSA. Sample containing target analyte is added to the sample well and incubated for 30 minutes. After incubation, these wells are washed 3 times with PBS containing 0.1 % TWEEN^{®} 20. Detection antibody labeled with fluorescer (100 µL, 10 µg/mL) is then added to each of the wells and incubated for 30 min at 37 °C. Then these wells are washed 3 times with PBS with 0.1% TWEEN^{®} 20. After washing off unbound detection antibody, chemiluminescent substrate containing TCPO, catalyst, and hydrogen peroxide is added to each well and the light intensity is measured with a plate reader in luminescent mode. The intensity of light is interpreted as indicating the amount of target analyte present.

In a modification, particles labeled with both antibody and fluorescer are used to detect the target analyte.

### Example 8: Detection of pathogen RNA using lateral-flow assay

In one example, the technology introduced here can be used for the detection of pathogen RNA in a body fluid sample. A sample is added to reverse transcriptase reaction mix and incubated for 10 to 30 minutes to generate cDNA. An aliquot of the generated cDNA is added to PCR master mix with specific primers labeled with non-intercalating fluorescer and subjected to temperature cycling from 25 to 45 cycles of annealing and denaturation to amplify the cDNA. Denaturation solution is added to the amplified sample, then mixed with running buffer and applied to the upstream end of a dry lateral-flow wicking matrix. In the preferred embodiment, the membrane is constructed from nitrocellulose on a polymer-based backing support material. The membrane is spotted with a test line for analyte detection, said line comprising sequence-specific DNA oligomer probes that complement the amplified target. The membrane is also spotted with a control line consisting of a molecular recognition moiety that binds specifically to the labeled primer. An absorbent pad is selected to effectively wick the liquid sample through the strip, and ideally prevent backflow, which can hamper sensitivity. After development, chemiluminescent substrate containing freshly prepared mixture of hydrogen peroxide and peroxyoxalate is added to the lateral-flow material (optionally from a blister or other storage volume associated with the LFA membrane). The lateral-flow strip is immediately imaged or scanned for the presence of light signal at the position of the second, capture, antibody using the camera or light detector.

In a modification, the reverse transcriptase reaction and the DNA amplification is carried out in one step.

In another modification, the DNA amplification reaction is an isothermal reaction such as recombinase polymerase amplification (RPA), loop mediated isothermal amplification (LAMP), or any isothermal amplification techniques.

### Example 9: Binding assay with optical fibers

In another example, target analyte can be detected and measured using a chemiluminescent signal detected with optical fibers. Sample containing target analyte is added to a reaction tube containing chemiluminescent particles bearing antibodies to target analyte. The optical fiber coated with antibody that recognizes the target analyte, and blocked with BSA, is introduced into a reaction tube. After incubation, the optical fiber is washed with PBS containing 0.1 % TWEEN^{®} 20. After washing, the optical fiber is introduced to chemiluminescent substrate. Light intensity is measured from the other end of the fiber. The intensity of light is interpreted as the amount of target analyte present.

In a modification, multiple optical fibers are used. In some applications, the optical fibers are bundled up into a single shaft. In other applications, the optical fibers are separated at the ends.

In another modification, the optical fibers are individually coated with different antibodies and numerous different detection antibodies conjugated to fluorescer are used for detecting multiple target analytes. In some application, multiple fluorescers are used to identify different target analytes.

In another modification, a photonic crystal fiber (PCF) is used instead of a traditional optical fiber. The functionalization and reaction are done in the hollow conduit of the PCF.

### Example 10: CRET assay on fiber tip

In another example, target analyte can be detected and measured by chemiluminescence resonance energy transfer (CRET) signal with optical fibers. Sample containing target analyte is added to a reaction tube containing chemiluminescent particles bearing antibodies to target analyte. The optical fiber is coated with antibody that recognizes the target analyte that is conjugated with a non-chemiexitable fluorescent dye. The optical fiber is blocked with BSA to prevent nonspecific interaction. The fiber is introduced into a reaction tube contain the sample mixed with chemiluminescent substrate. Light intensity is measured from the other end of the fiber with optical filter allowing only the signal from non-chemiexitable fluorescent dye to pass through. The intensity of light is interpreted as indicating the amount of target analyte present.

In another modification, a photonic crystal fiber (PCF) is used instead of traditional optical fiber. The functionalization and the reaction are done in the hollow conduit of the PCF.

### Example 11: Detection of protein using flotation assay.

In an example, target analyte can be detected and measured in a chemiluminescent flotation assay. Glass microbubbles are coated with antibody that recognizes the target analyte using silane chemistry and blocked with BSA. Sample containing target analyte is added to the reaction tube containing the microbubbles and chemiluminescent reporter particles. The reaction tube is designed to have an optically clear top with conical shape for concentrating the microbubbles. The reaction tube is incubated for up to 30 minutes on a rotator. After incubation, chemiluminescent substrate containing TCPO, hydrogen peroxide, and other additives in a non-toxic organic solvent such as ethyl acetate is added to the reaction tube so that the conical top is filled with liquid. In the absence of target analyte, the chemiluminescent reporters are in the bottom aqueous phase and not interacting with the chemiluminescent substrate, thus producing no or low light signal. In the presence of the target analyte, the chemiluminescent reporters are carried to the top by microbubbles in the sandwich complex of antibody-target analyte-antibody conjugated to reporter. The light intensity is read at the top of the reactor tube. The intensity of light is interpreted as the amount of target analyte present in the sample.

### Example 12: Detection of protein using magnetic beads

In another example, target analyte can be detected and measured in a chemiluminescent assay with magnetic beads. The iron oxide magnetic beads are coated with antibody that recognizes the target analyte using silane chemistry and blocked with BSA. Sample containing target analyte is added to the reaction tube containing the magnetic beads and chemiluminescent reporter particles. The reaction tube is incubated for up to 30 minutes on a rotator. After incubation, a magnet placed on the side of the tube to collect the magnetic beads. Liquid is removed by pipetting without disturbing the magnetic beads collected on the side. The magnetic beads are washed 3 times with PBS, 0.1 % TWEEN^{®} 20. After washing, beads are collected to the side again with a magnet. Liquid is then removed by pipetting leaving only magnetic beads. (These steps optionally can be automated by a robotic or microfluidic liquid handling device). Chemiluminescent substrate containing TCPO, hydrogen peroxide, and other additives in a organic solvent such as ethyl acetate or surfactant-containing water is mixed into the reaction tube. In the presence of the target analyte, the chemiluminescent reporters are retained by the magnetic beads in the sandwich complex of antibody-target analyte-antibody conjugated to reporter. The light intensity is measured with a luminometer. The intensity of light is interpreted as the amount of target analyte present in the sample.

### Example 13: Detection of protein using microfluidic device

In an example, the sample is applied to a microfluidic device in which includes a assay chamber that is functionalized with capture antibody, a detection reagent chamber containing particles functionalized with antibody and fluorescer, and development reagent chambers. The sample flows through the detection reagent chamber to resuspend the particles and to allow the target molecules to react with the particles. Then the mixture is pumped to the assay chamber in which the particle, the target, and the capture antibody react and form the sandwich complex. Wash buffer is then flowed through the assay chamber to wash away the unbound particles. After washing, the reagents from development chambers are mixed and pumped to the assay chamber. The light intensity is measured with a luminometer. The intensity of light is interpreted as indicating the amount of target analyte present in the sample.

### Example 14: Continuous detection in flow

In an example, a fluorescently-labeled binding ligand *(e.g.,* an antibody modified with fluorescein) able to recognize and bind to a molecule is continuously added to a smaller sidestream of the effluent of a large preparative chromatography column of, *e.g.,* 20 L in volume. Capture ligands recognizing the same target are immobilized on a surface, and chemiexcitation reagents are added to the sidestream. When the target recognized by the capture ligands begins to emerge from the column, it causes accumulation of the fluor-labeled detector on the capture ligands by sandwich capture. The resulting increase in florescence emission from the capture surface is detected by measuring light intensity, optionally and preferably without a wavelength filter between the light detector and the capture surface.

### Example 15: Spintharoscopic detection

In an example, excitation is by low levels of radioactive decay, with detection by a phosphor or scintillator. The quantity of radioactive material used is low enough to be exempt from stringent regulatory constraints, e.g. 0.1, 0.001, or 0.00001 microCurie, and detection is by imaging of the emissions resulting from single radioactive decay events, as in a spintharascope with digital electronic imaging. Particles smaller than 200 nm, having less than 0.05% uranium or thorium by weight and decorated with detection antibodies recognizing an analyte, can be used with a scintillator screen decorated with capture antibodies against that analyte.

### Example 16: Detection and quantification of norovirus by counting chemiexcited particle immunoagglutinates

In an example, norovirus detection is performed on a porous membrane by imaging and counting immunoagglutination of chemiexcited fluorescent particles induced by norovirus. A norovirus containing sample (5 µL) is added to the center of a nitrocellulose porous membrane. After loading the sample, 5 µL of a solution of anti-norovirus-antibody-conjugated fluorescent polystyrene particles is loaded onto the center of the nitrocellulose porous membrane where noroviruses were loaded. In the presence of norovirus, the fluorescent particles are aggregated by immunoagglutination. To develop the signal, 200 microliters of a solution of oxalate ester and hydrogen peroxide is applied to the strip, which is then imaged with a microscope attached to a camera in a dark box. The chemiexcitation of the fluors on the aggregated virus particles produces bright emission spots. An image-processing algorithm counts the aggregated particles to determine the presence and amount of the norovirus particles.

In a modification, the norovirus containing sample is mixed with anti-norovirusconjugated fluorescent polystyrene particles before adding to the membrane.

### Example 17: Short LFA for fast HIV detection

In an example, a fast lateral flow sandwich immunoassay is conducted on a short nitrocellulose strip, < 1 cm in length between sample pad and absorbent pad, to detect HIV core antigen p24. The reporter particles bearing the detector antibody are fluorescent, and are loaded with an organic fluorescent molecule to such a high concentration that self-quenching of the fluors occurs to more than 10%. A human plasma sample is mixed with fluorescent nanoparticles bearing an antibody recognizing an epitope of p24. The mixture is flowed along a short nitrocellulose LFA membrane with a test line containing an antibody recognizing another epitope of HIV p24 and anti-species control line. After the sample has been run, 100 microliters of a mixture of oxalate ester in butyl benzoate and hydrogen peroxide in t-butanol containing an agent which increases viscosity to at least 3 cPoise is applied to the strip. Fluorescent molecules are leached from the particles and chemically excited, and then imaged with a cell phone camera fitted with a lens in a dark box. The chemiexcitation of the fluors on the particle produces a bright emission at the test line, which is numerically integrated and divided by the emission intensity at the control line to estimate the concentration of p24 in the original sample. The lens is designed with the capability to resolve the test and control lines.

### Example 18: Syringe-driven flow in a packed bed of microparticles for fast Ricin detection

In an example, a fast lateral-flow sandwich immunoassay is conducted on a translucent packed bed column of microparticles with flow pressure-driven by syringe to detect ricin. The reporter particles bearing the detector antibody comprise fluorescent molecules. A diluted sample is drawn into a syringe and mixed with a preloaded suspension of fluorescent nanoparticles bearing an antibody recognizing an epitope of one chain of ricin. The syringe is also preloaded with a wash buffer separated by an air gap (optionally, the detector and wash reagents may alternately be in two immiscible aqueous two-phase liquids). Oxalate ester solution and hydrogen peroxide solution are also stored in breakable compartments separately in the syringe, or a separate syringe. The sample-particles mixture is pumped through a packed bed of microparticles functionalized with an antibody recognizing another epitope of ricin. After the sample has been pumped through, the plunger breaks compartments containing a solution of oxalate ester and hydrogen peroxide, allowing the two solutions to mix as they are pumped onto the packed bed. The translucent packed bed column is then imaged with a cell phone camera fitted with a lens and draped with dark fabric. The chemiexcitation of the particle fluors produces a bright emission at the test line, which is displayed on the phone screen and optionally numerically integrated to estimate the concentration of ricin in the original sample.

### Example 19: TCPO and fluorophore-embedded polystyrene particles as LFA reporter particles

In an example, TCPO is embedded into polystyrene particles along with fluorescent dye to simplify the chemiexcitation reagent to only one component, thus eliminating the mixing step. TCPO and fluorescent dye are dissolved in an organic solvent. The mixture is added to a 1% solid polystyrene particle suspension with a final concentration of the organic solvent less than 30%. Upon the addition of organic solvent, the polystyrene particles swell and allow TCPO and fluorescent dye to diffuse into the particles. Adding surfactant in the particle suspension can reduce aggregation of particles. After incubation, the suspension mixture is thoroughly washed several times by centrifugation or dialysis to remove unincorporated TCPO and dye. The TCPO fluorescent particles are functionalized with a detector antibody and used as reporter particles in an LFA format. A human plasma sample is mixed with fluorescent nanoparticles bearing an antibody recognizing an epitope of p24. The mixture flows along a nitrocellulose LFA membrane with a test line containing an antibody recognizing another epitope of HIV p24 and anti-species control line. After the sample has been run, 100 microliters of a solution of containing hydrogen peroxide is applied to the strip, which is then imaged with a cell phone camera fitted with a lens in a dark box. The chemiexcitation of the fluors on the particle produces a bright emission at the test line, which is numerically integrated and divided by the emission at the control line to estimate the concentration of p24 in the original sample.

In a modification, hydrogen peroxide is added to the LFA running buffer to trigger continuous optical emission from the particles.

### Example 20: LFA cassette with built-in reagent pouches and channels for storing, mixing, and delivering TCPO and hydrogen peroxide reagents

In an example, the LFA cassette has reagent pouches and channels made of thermoplastic film (fluorinated ethylene propylene (FEP) or perfluoroalkoxy (PFA)). These fluorinated plastics are highly inert and compatible with most organic compounds. The reagent pouches and channels will be produced by a scalable thermoforming process. The reagent delivery system consists of two pouches, <100 microliters capacity, connected by a channel. Another channel connects a reagent pouch to the LFA strip. Reagents will be retained in the pouches by pinching pressure applied to the connecting channels by breakaway levers. The entire reagent pouches, channels and valves are fitted in LFA cassette with less than 1 inch in width and 4 inches in length. Beside reagent storage stability, the cassette is designed to mix and dispense reagents in 4 steps. The user adds 50 microliters of the sample to the well and allows the LFA to run for <15 minutes. Signal generation takes 4 steps: flipping a first valve lever to connect the two reagent pouches, alternately pressing on the two reagent pouches to mix for <5 times, flipping the second valve lever, and finally pressing on both pouches to dispense the chemiexcitation mixture onto the LFA lines.

### Example 21: Competitive assay to detect tacrolimus in blood

In an example, a small drug molecule such as tacrolimus is detected on a single LFA strip in competitive assay format. 50 microliters of human fingerstick blood is applied to the membrane through a Vivid blood filter that is overlapped with the conjugate release pad containing fluorescent nanoparticles decorated with tacrolimus-BSA conjugate. The mixture flows along a nitrocellulose LFA membrane with an anti-tacrolimus test line and anti-BSA control line. After the sample has been run, 100 microliters of a solution of oxalate ester and hydrogen peroxide is applied to the strip, and brightness at each strip is detected by individual photodiodes. Light emission at the test line is divided by the emission at the control line to estimate the concentration of tacrolimus in the original sample. In a modification, the tacrolimus-BSA conjugate is on the test line and the particles are decorated with anti-tacrolimus antibodies.

### Example 22: CRET Competitive assay to detect tacrolimus

In an example, small drug molecule such as tacrolimus is detected in a sample well in competitive assay format with chemiluminescent resonance energy transfer (CRET) signal readout. 50 microliters of human plasma is mixed with cyclam-tacrolimus conjugate in a sample well containing small (diameter < 20 nm) 9,10-diphenylanthracene (DPA) fluorescent nanoparticles decorated with anti-tacrolimus antibody with Cu₂₊ containing buffer. After incubation, 100 microliters of a solution of oxalate ester and hydrogen peroxide is applied to the well, which is then imaged with a cell phone camera in a dark box. In the absence of tacrolimus, the tacrolimus-cyclam-Cu₂₊ complexes bind to the anti-tacrolimus antibodies on the DPA fluorescent nanoparticles and quench the light emission. When free tacrolimus is present, the free tacrolimus molecules from the plasma sample compete with tacrolimus-cyclam-Cu₂₊ complexes and remove the quenching effect resulting in bright emission. The intensity of light emission is used to estimate the concentration of tacrolimus in the original sample.

### Example 23: DNA fluor probes-quencher

In an exemplary embodiment, a sample containing target DNA is added to a polymerase chain reaction (PCR) master mix containing fluor-labeled probe and a primer pair that is specific to the target DNA. Optionally, the PCR master mix also contains a non-intercalating fluorescer such as ROX reference dye. The fluor-labeled probe is constructed containing a fluorescent reporter dye on the 5' end and a quencher dye on the 3' end. The probe is designed so that the proximity of the quencher greatly reduces the emission of the fluor by fluorescence resonance energy transfer (FRET). The 3' end may contain a minor-groove binder. The PCR polymerase is chosen to have 5' nuclease activity. After PCR reaction, 50 microliters of a solution of oxalate ester and hydrogen peroxide is applied to the strip, which is then imaged with a cell phone camera fitted with a lens in a dark box. The presence of the target DNA produces bright emission due to the cleaved probe. The intensity is proportional to the amount of amplicon produced.

In a modification, the PCR is replaced by an isothermal amplification such as recombinase polymerase amplification (RPA). In another modification, solution of oxalate ester and hydrogen peroxide is added before PCR reaction, and light intensity measurement is performed during the reaction.

### Example 24: LFA on plate

In an example, a protein target is detected on a small LFA membrane in a microtiter well format. Short membrane strips that are constructed with the same components and design as a typical LFA strip are carefully fitted inside the wells of the well plate. Each well is divided into three compartments with each contains a portion of the LFA strip (sample pad, assay membrane, and absorbent pad). The compartments are sealed to only allow liquid to travel between the compartments by flowing through the porous membrane. The strips are striped or spotted with antibody that recognizes the target analyte. Samples are mixed with fluorescent nanoparticles bearing an antibody recognizing another epitope of the analyte are added to the compartments of the wells containing sample pads. Samples with known concentrations of the analyte can be run on the same well plate to serve as standards. The mixture is flowed along a short nitrocellulose LFA membrane and encounters the antibody on the membrane. After the sample has been run, 100 microliters of a solution of oxalate ester and hydrogen peroxide is applied to the middle compartment containing the assaying part of the strip. Light intensity is measured with a plate reader in luminescent mode. The intensity of light is compared with the intensities from the standards to calculate the amount of target analyte present.

### Example 25: Multiplexing with different color particles in LFA to detect multiple analytes

In an example, biowarfare agents (BWAs) such as staphylococcus enterotoxin B, ricin, and botulinum toxin are detected simultaneously on a single multiplex LFA strip. The suspected sample is mixed with a mixture of different-fluor particles. Each fluorescent color particle bears a different antibody recognizing an epitope of a toxin (*i.e.,* blue fluorescer particles are functionalized with anti-staphylococcus enterotoxin B, green fluorescer particles are functionalized with anti-ricin, and red fluorescer particles are functionalized with antibotulinum toxin). The mixture flows along a nitrocellulose LFA membrane with a test line containing a mixture of antibodies recognizing other epitopes of the toxins, or multiple test lines with different antibodies. The control line is functionalized with a mixture of anti-species antibodies recognizing the antibodies on the particles. After the sample has been run, 200 microliters of a solution of oxalate ester and hydrogen peroxide is applied to the strip, which is then imaged with a cell phone camera fitted with a lens in a dark box. The chemiexcitation of the fluors on the particle produces a bright emission at the test line, which is displayed and also optionally RGB split, numerically integrated, and divided by the emission at the control line to estimate the concentration of toxin in the original sample. The three RGB color channels of a camera provide immediate 3-analyte multiplexing with a single excitation liquid and different-fluor particles, and LFAs with 2 or 3 lines can be used to increase the number of analytes further.

### Example 26: CRET assay to detect metal ions

In an example, metal ion such as Cu₂₊ is detected in a sample well with chemiluminescent resonance energy transfer (CRET) signal readout. 50 microliters of sample is mixed with small (diameter < 20 nm) polystyrene particles dyed with 9,10-diphenylanthracene (DPA) as the donor and pyromethene 567 (PM567) as the acceptor. The particles are also grafted with cyclam to bind to Cu₂₊. After incubation, 100 microliters of a solution of oxalate ester and hydrogen peroxide is added to the sample, which is then imaged with a cell phone camera in a dark box. In the presence of Cu₂₊, the cyclam-Cu₂₊ complexes quench the light emission. The intensity of light emission is used to estimate the concentration of Cu₂₊ in the original sample.

### Example 27: Nucleic acid detection with DNA molecular beacons

In an exemplary embodiment, a sample containing target DNA is added to a sample well containing hairpin shaped molecules with a fluorescent reporter dye on one end, a probe sequence that is specific to the target DNA in the middle, and a quencher dye on the other end. A few bases (about 5 to 7 nucleotide residues) on the termini are complementary to each other to hold the fluorescent reporter dye and the quencher in close proximity. After hybridization of the molecular beacon to the target, 50 microliters of a solution of oxalate ester and hydrogen peroxide is added to the well, which is then read with a luminometer or imaged with a cell phone camera fitted with a lens in a dark box. In the absence of the target DNA, the probe is in the hairpin conformation, quenching the chemi-excited fluorescent reporter. Upon binding to a target nucleic acid sequence, the chemi-excited fluorescence of the probe is higher. The intensity is proportional to the amount of target DNA present.

### Example 28: Reel of LFA ribbon for continuous toxic and pathogen detection

In an example, a pathogen is continuously monitored with a reel of LFA ribbon. The ribbon is striped with a test line containing an antibody recognizing epitope of the pathogen and an anti-species control line along the ribbon length. One edge of the ribbon serves as the absorbent pad, the other edge contains a sample pad overlapping a conjugate release pad. Optionally the ribbon is of uniform composition, *e.g*., silica fibers, with conjugate, test and capture lines deposited. The conjugate release pad contains fluorescent reporter particles bearing the detector antibody. The ribbon is segmented with lines across its width by of a water barrier compound such as wax, thus effectively creating multiple separate LFA strips in a reel. 50 microliters of sample is injected onto the sample pad area of a segment. The sample is mixed with fluorescent nanoparticles as it flows along the LFA membrane (across the ribbon). After a determined time duration or ribbon travel distance, 50 microliters of a solution of oxalate ester and hydrogen peroxide is applied to the segment, which is then read with a camera or light detector fitted in a dark box. In presence of the target analyte, the chemiexcitation of the fluors on the particle produces a bright emission at the test line, which is numerically integrated and divided by the emission at the control line to estimate the concentration of the analyte in the original sample. The ribbon is continuously or occasionally unwound to access new LFA strip segments from a reel and the used segments are wound up on another reel.

In a modification, the toxin is monitored with a reel of LFA ribbon in a competitive assay format. 50 microliters of sample is timely injected onto the sample pad of a segment. The sample flows through the conjugate release pad and mixes with fluorescent nanoparticles decorated with toxin-BSA conjugate. The mixture flows along a nitrocellulose LFA membrane with an anti-toxin test line and anti-BSA control line. After the sample has been run, 50 microliters of a solution of oxalate ester and hydrogen peroxide is applied to the segment, which is then read with a camera or light detector fitted in a dark box. In presence of the target toxin, the chemiexcitation of the fluors on the particle produces a bright emission at the test line, which is numerically integrated and divided by the emission at the control line to estimate the concentration of the toxin in the original sample.

In a modification, the LFA ribbon is made without an adhesive backing layer and is read on the side of clear plastic backing of the membrane. In another modification, the LFA ribbon is detected by conventional fluorescence reading, or imaging of colored particles.

### Example 29: Spool of LFA thread for continuous toxin and pathogen detection

In an example, a pathogen is repeatedly monitored with a spool of LFA thread. The thread is dipped, sprayed or blotted at fixed length intervals with a solvent resistant, water barrier compound to create many short non-intercommunicating segments along the thread in a spool. Each segment of the thread has a test spot containing an antibody recognizing an epitope of the pathogen and an anti-species control spot. One end of each segment contains fluorescent reporter particles bearing the detector antibody. The thread is partially unwound to make a segment accessible for use. Ten microliters of sample is injected onto one end of the segment containing the reporter particles. The sample is mixed with fluorescent nanoparticles flows along the LFA thread. After a predetermined time or thread travel distance, 10 microliters of a solution of oxalate ester and hydrogen peroxide is applied to the segment, which is then read with a camera, photodiode, PMT, or light detector fitted in a dark box. In presence of the target analyte, the chemiexcitation of the fluors on the particle produces a bright emission at the test spot, which is numerically integrated and divided by the emission at the control spot to estimate the concentration of the analyte in the original sample. The thread is continuously unwound out new LFA segments from a spool and the used segments are wound up in another spool.

In a modification, the toxin is monitored with a reel of LFA thread in a competitive assay format. 10 microliters of sample is timely injected onto one end of the segment containing the reporter particles. The sample flows through the conjugate release pad and mixes with fluorescent nanoparticles decorated with toxin-BSA conjugate. The mixture flows along a nitrocellulose LFA membrane with an anti-toxin test line and anti-BSA control line. After the sample has been run, 50 microliters of a solution of oxalate ester and hydrogen peroxide is applied to the segment, which is then read with a camera or light detector fitted in a dark box. In presence of the target toxin, the chemiexcitation of the fluors on the particle produces a bright emission at the test spot, which is reported and optionally numerically integrated and divided by the emission at the control spot to estimate the concentration of the toxin in the original sample.

In another modification, the thread LFA results are detected by conventional fluorescence reading, or imaging of colored particles.

### Example 30: Detection of protein target by sandwich binding assay in solution with antibody-oxidase conjugate and fluorescent particles

In another example, target analyte can be detected and measured in a chemiluminescent immunosorbent assay. Fluorescent, TCPO-embedded nanoparticles are coated with antibody recognizing an epitope of the target analyte and blocked with BSA. Another antibody recognizing a different epitope of the target analyte is conjugated with glucose oxidase. Samples are added to the sample well plate containing the fluorescent particles and antibody-glucose oxidase conjugate. After incubation, developing solution containing glucose, and catalase is added to each well and the light intensity is measured with a plate reader in luminescent mode. In the absence of the analyte, the hydrogen peroxide produced by glucose oxidase is decomposed by catalase, thus chemiluminescence occurs only weakly. In the presence of the analyte, immunocomplexes are formed between the antibody-glucose oxidase conjugates and fluorescent particles allowing the hydrogen peroxide to react with the particles due to its close proximity to the particles, allowing it to avoid decomposition. The intensity of light is interpreted as indicating the amount of target analyte present.

### Example 31: Chemiexcitation of LFA flour labeled antibody for sensitive imaging detection

A lateral flow sandwich immunoassay is conducted on a nitrocellulose strip with a sandwich pair of antibodies recognizing HIV core antigen p24. The detector antibody are labeled with fluorescent dye to serve as a reporter. The detector antibody recognizes an epitope of HIV p24. A human plasma sample is mixed with the detector antibody and flowed along a nitrocellulose LFA membrane with a test line bearing a different antibody recognizing a second epitope of p24 and anti-species control line. After the sample has been run, 100 microliters of a solution of oxalate ester and hydrogen peroxide is applied to the strip, which is then imaged with a cell phone camera in a dark box. The chemiexcitation of the fluors on the particle produces a bright emission at the test line, which is numerically integrated and divided by the emission at the control line to estimate the concentration of p24 in the original sample.

In a modification, after running, the strip is scanned with a light detector mounted on a linear actuator.

### Example 32: Nucleic acid detection with surface-enhanced chemi-excitation on nanoporous gold disk plasmonic nanoparticles

In an exemplary embodiment, a sample containing target DNA is added to a sample well containing hairpin shaped molecules with a fluorescent reporter dye on one end, a probe sequence that is specific to the target DNA in the middle, and a nanoporous gold disk plasmonic nanoparticles. A few bases (about 5 to 7 nucleotide residues) on the termini are complementary to each other to hold the fluorescent reporter dye and the nanoporous gold disk plasmonic nanoparticles in close proximity. After hybridization of the molecular beacon to the target, 50 microliters of a solution of oxalate ester and hydrogen peroxide is added to the well, which is then read with a luminometer or imaged with a cell phone camera fitted with a lens in a dark box. In the absence of the target DNA, the probe is in the hairpin conformation, quenching the chemi-excited fluorescent reporter. Upon binding to a target nucleic acid sequence, the chemiexcited fluorescence of the probe is higher. The intensity is proportional to the amount of target DNA present.

## Claims

1. A method of performing a lateral or vertical flow assay comprising (a) providing a fluid sample comprising an analyte of interest; (b) contacting said sample with a membrane or filter comprising a first reagent comprising a molecular recognition element that binds to said analyte; (c) contacting said sample with a second reagent that:
(i) binds to said analyte in the presence of said first reagent; or
(ii) binds to said first reagent;
wherein said second reagent contains a chemiexcitation emitter; (d) removing unbound first and second reagents; (e) introducing into said sample a chemiexcitation stimulator; and (f) measuring the production of light from said emitter,
wherein the chemiexcitation stimulator is peroxyoxalate chemiluminescence resulting from the energy transferred from the reaction of peroxide and peroxyoxalate ester to the chemiluminescent emitter, and
wherein the second reagent is in the form of a particle coated or functionalized with the chemiexcitation emitter and the recognition element.

2. The method of claim 1, wherein the molecular recognition element comprises an aptamer, a protein, a carbohydrate or a nucleic acid, such as a protein beacon, analyte-sensitive fluorescent protein, allosteric molecular beacon, hairpin peptide beacon, molecular aptamer beacon, quantum dot aptamer beacon, molecular beacon aptamer, antibody, allosteric fluorescent protein biosensor, or molecular switch sensor.

3. The method of claim 1, further comprising introducing a catalyst into the sample, such as wherein the catalyst is sodium salicylate, tripropylamine, triethylamine, sodium acetate, tetrabutylammonium perchlorate, benzyltrimethylammonium hydroxide, or oxalic, trichloroacetic, or picric acid.

4. The method of claim 1, wherein the chemiluminescent emitter comprises at least one chemiluminescent particle and at least one magnetic particle or layer of magnetic material, such as wherein a shell encapsulates the at least one chemiluminescent particle and the at least one magnetic particle or layer of magnetic material.

5. The method of claim 4, further comprising concentrating the magnetic chemiluminescent emitter by applying a magnetic field.

6. The method of claim 1, wherein said method is a quantitative assay.

## Patentansprüche

1. Ein Verfahren zur Durchführung eines lateralen oder vertikalen Durchfluss-Assays umfassend (a) Bereitstellen einer flüssigen Probe umfassend einen Analyten von Interesse; (b) Kontaktieren der Probe mit einer Membran oder einem Filter umfassend ein erstes Reagenz umfassend eine molekulares Erkennungselement, das an den Analyten bindet; (c) Kontaktieren der Probe mit einem zweiten Reagenz, das:
(i) an den Analyten in Gegenwart des ersten Reagenz bindet; oder
(ii) an das erste Reagenz bindet;
wobei das zweite Reagenz einen chemischen Erregungsemitter enthält; (d) Entfernen der ungebundenen ersten und zweiten Reagenzien; (e) Einbringen eines chemischen Erregungsstimulators in die Probe und (f) Messen der Lichtproduktion des Emitters,
wobei der chemische Erregungsstimulator Peroxyoxalat-Chemilumineszenz ist, die aus der Energie resultiert, die durch die Reaktion von Peroxid und Peroxyoxalatester auf den Chemilumineszenz-Emitter übertragen wird und
wobei das zweite Reagenz in der Form eines Partikels ist, der mit dem chemischen Erregungsemitter und dem Erkennungselement beschichtet und funktionalisiert ist.

2. Das Verfahren nach Anspruch 1, wobei das molekulare Erkennungselement ein Aptamer, ein Protein, ein Kohlenhydrat oder eine Nukleinsäure, wie beispielsweise ein Protein-Beacon, Analyten-sensitives fluoreszierendes Protein, allosterisches Molecular Beacon, Haarnadelpeptid-Beacon, Molecular Aptamer-Beacon, Quantenpunkt-Aptamer-Beacon, Molecular Beacon-Aptamer, Antikörper, allosterischer fluoreszierender Protein-Biosensor oder molekularer Schaltersensor umfasst.

3. Das Verfahren nach Anspruch 1, ferner umfassend Einbringen eines Katalysators in die Probe, wobei der Katalysator beispielsweise Natrium-Salicylat, Tripropylamin, Triethylamin, Natriumacetat, Tetrabutylammoniumperchlorat, Benzyltrimethylammoniumhydroxid oder Oxalsäure, Trichloressigsäure oder Pikrinsäure ist.

4. Das Verfahren nach Anspruch 1, wobei der Chemilumineszenz-Emitter mindestens einen Chemilumineszenz-Partikel und mindestens einen magnetischen Partikel oder Schicht eines magnetischen Materials umfasst, wobei beispielsweise eine Hülle den mindestens einen Chemilumineszenz-Partikel und den mindestens einen magnetischen Partikel oder Schicht eines magnetischen Materials umschließt.

5. Das Verfahren nach Anspruch 4, ferner umfassend Konzentrieren des magnetischen Chemilumineszenz-Emitters durch Anlegen eines Magnetfeldes.

6. Das Verfahren nach Anspruch 1, wobei das Verfahren ein quantitativer Assay ist.

## Revendications

1. Méthode de mise en œuvre d'un dosage à écoulement latéral ou vertical, comprenant (a) la fourniture d'un échantillon de fluide comprenant un analyte d'intérêt ; (b) la mise en contact dudit échantillon avec une membrane ou un filtre comprenant un premier réactif comprenant un élément de reconnaissance moléculaire qui se lie audit analyte ; (c) la mise en contact dudit échantillon avec un deuxième réactif qui :
(i) se lie audit analyte en présence dudit premier réactif ; ou
(ii) se lie audit premier réactif ;
dans laquelle ledit deuxième réactif contient un émetteur de chimio-excitation ;
(d) l'élimination des premier et deuxième réactifs non liés ; (e) l'introduction dans ledit échantillon d'un stimulateur de chimio-excitation ; et (f) la mesure de la production de lumière provenant dudit émetteur,
dans laquelle le stimulateur de chimio-excitation est la chimioluminescence d'un peroxyoxalate résultant de l'énergie transférée à partir de la réaction de peroxyde et d'ester peroxyoxalate à l'émetteur chimioluminescent, et
dans laquelle le deuxième réactif est sous la forme d'une particule revêtue ou fonctionnalisée avec l'émetteur de chimio-excitation et l'élément de reconnaissance.

2. Méthode selon la revendication 1, dans laquelle l'élément de reconnaissance moléculaire comprend un aptamère, une protéine, un hydrate de carbone ou un acide nucléique, tel qu'une balise protéique, une protéine fluorescente sensible à un analyte, une balise moléculaire allostérique, une balise peptidique en épingle à cheveux, une balise aptamère moléculaire, une balise aptamère à boîte quantique, un aptamère de balise moléculaire, un anticorps, un biocapteur protéique fluorescent allostérique, ou un capteur de commutation moléculaire.

3. Méthode selon la revendication 1, comprenant en outre l'introduction d'un catalyseur dans l'échantillon, telle que dans laquelle le catalyseur est le salicylate de sodium, la tripropylamine, la triéthylamine, l'acétate de sodium, le perchlorate de tétrabutylammonium, l'hydroxyde de benzyltriméthylammonium, ou l'acide oxalique, trichloroacétique, ou picrique.

4. Méthode selon la revendication 1, dans laquelle l'émetteur chimioluminescent comprend au moins une particule chimioluminescente et au moins une particule magnétique ou couche de matériau magnétique, telle que dans laquelle une gaine encapsule l'au moins une particule chimioluminescente et l'au moins une particule magnétique ou couche de matériau magnétique.

5. Méthode selon la revendication 4, comprenant en outre la concentration de l'émetteur chimioluminescent magnétique par application d'un champ magnétique.

6. Méthode selon la revendication 1, laquelle méthode est un dosage quantitatif.
